# EUROPEAN PATENT APPLICATION

(11) **EP 3 144 381 A1**
(43) Date of publication of application: **22.03.2017**
(21) Application number: 15792643.7
(22) Date of filing: 15.05.2015
(51) Int. Cl.: C12N 1/20, A01H 1/00, A01H 5/00, C12N 1/21, C12N 15/09

(54) **AGROBACTERIUM AND TRANSGENIC PLANT MANUFACTURING METHOD USING SUCH AGROBACTERIUM**

(30) Priority: 16.05.2014 JP 2014102566
(71) Applicant: Kaneka Corporation, Osaka-shi, Osaka 530-8288 (JP)
(72) Inventor: HAMADA Haruyasu, Takasago-shi Hyogo 676-8688 (JP); NAGIRA Yozo, Takasago-shi Hyogo 676-8688 (JP); TAOKA Naoaki, Takasago-shi Hyogo 676-8688 (JP); IMAI Ryozo, Sapporo-shi Hokkaido 062-8555 (JP); KOJIMA Mineo, Nagoya-shi Aichi 465-0053 (JP); MIKI Ryuji, Takasago-shi Hyogo 676-8688 (JP)
(74) Representative: Hoffmann Eitle
(86) International application number: PCT/JP2015/063990
(87) International publication number: WO 2015/174514

(57) **Abstract**

It is an object solved by the present invention to provide an Agrobacterium with increased gene targeting efficiency. The present invention provides an Agrobacterium having a T-DNA nuclear translocation ability equivalent to that of a wild-type Agrobacterium and also having a T-DNA chromosomal insertion ability that is lost or reduced in comparison to a wild-type Agrobacterium.

## Description

### Technical Field

The present invention relates to Agrobacterium with increased gene targeting efficiency, and a method for producing a transformed plant using the same.

### Background Art

In the currently popular plant transformation technique (Non Patent Literature 1), a gene of interest cannot be inserted into a targeted site on the chromosome, but it can be randomly inserted into a site on the chromosome. In addition, the copy number of genes of interest to be introduced cannot be regulated. Thereby, the current transformation technique is problematic, for example, in that a variation in the gene expression level depending on the insertion site of a foreign gene, namely, "position effect" is generated (Non Patent Literature 2), in that a gene of interest is inserted into a position on the chromosome that affects normal growth, and in that insertion of a plurality of genes of interest causes inactivation of the genes of interest. Hence, in the current transformation technique, it is necessary to produce an enormous number of transformants, and then to select transformants, in which a gene of interest has been inserted into only a desired site. Thus, the current transformation technique is problematic in terms of poor efficiency. In order to overcome this problem and to produce safe and highly-functional, genetically-modified crops, it has been desired to develop a gene targeting technique capable of introducing a desired copy number of genes of interest into designated positions on the chromosome.

In recent years, as gene targeting techniques in plants that have been developed, methods of utilizing artificial nuclease, modification of a chromatin structure, or homologous recombination-related factors and the like have been reported (Non Patent Literature 3 to 5). These are all techniques of modifying a plant body to improve homologous recombination efficiency, and are not highly versatile in that optimization is required for each plant species to which the techniques are applied. Moreover, these techniques are also problematic in that the combined use with other techniques is difficult.

On the other hand, a Vir gene has been known as an Agrobacterium gene associated with insertion of a gene of interest into a site on the plant chromosome in the transformation of a plant using Agrobacterium. Patent Literature 1 discloses a method of allowing a protein to transiently express in a plant, and this publication also describes that a wild-type Agrobacterium strain (e.g., Agrobacterium tumefaciens, etc.), or an Agrobacterium strain in which one or several genes are mutated and transformation efficiency is thereby increased (e.g., an Agrobacterium strain comprising redundant virG gene copies, such as a pTiBo542-derived super virG gene preferably linked to a multiple-copy plasmid, such as, for example, an Agrobacterium strain, in which vir gene expression and/or induction thereof are modified by the presence of a mutant or chimeric virA gene or virG gene), is used as Agrobacterium (paragraph [0127]). Patent Literature 2 describes the use of an Agrobacterium strain comprising T-DNA containing a gene encoding a functional virE2 protein and a desired gene, together with an Agrobacterium strain comprising T-DNA containing a selective marker gene and having no ability to generate a functional virE2 protein, for simultaneous transformation of plant cells and the above-described two T-DNAs (Claim 10). Moreover, Patent Literature 3 discloses that the T-DNA delivery efficiency of an Agrobacterium deleting virE2 is approximately 10,000 times lower than that of a wild-type Agrobacterium or a mutant Agrobacterium supplemented with trans isomers (paragraph [0069]).

### Prior Art Literatures

### Patent Literatures

Patent Literature 1: JP Patent Publication (Kohyo) No. 2013-534430 A
Patent Literature 2: JP Patent Publication (Kohyo) No. 2011-505806 A
Patent Literature 3: JP Patent Publication (Kohyo) No. 2008-505622 A

### Non Patent Literatures

Non Patent Literature 1: Pitzschke and Hirt. New insights into an old story: Agrobacterium-induced tumor formation in plants by plant transformation. The EMBO Journal (2010) 29, 1021-1032
Non Patent Literature 2: Matzke A. and Matzke M. (1998) Position effects and epigenetic silencing of plant transgenes, Curr. Opin. Plant Biol. 1 142-148.
Non Patent Literature 3: Kumar et al. "Gene Targeting: Development of Novel Systems for Genome Engineering in Plants" Floriculture, Ornamental and Plant Biotechnology Volume IV (2006) Global Science Books
Non Patent Literature 4: Shukla, et al." Precise genome modification in the crop species Zea mays using zinc-finger nucleases." Nature (2009) 459 437-441
Non Patent Literature 5: Endo, et al. Increased frequency of homologous recombination and T-DNA integration in Arabidopsis CAF-1 mutants, EMBO J. 25 (2006) 5579-5590.

### Summary of Invention

### Object to be Solved by the Invention

It is an object solved by the present invention to provide a plant transformation technique that is highly versatile and can be used in combination with other plant transformation techniques. More specifically, it is an object solved by the present invention to provide an Agrobacterium with increased gene targeting efficiency. It is another object solved by the present invention to provide a method for producing the aforementioned Agrobacterium, plant cells obtained using the aforementioned Agrobacterium, and a method for producing a transformed plant using the aforementioned Agrobacterium.

### Means for Solving the Object

As a result of intensive studies directed towards achieving the aforementioned objects, the present inventors have found that gene targeting efficiency can be significantly increased by transforming a plant with an Agrobacterium having a T-DNA nuclear translocation ability equivalent to that of a wild-type Agrobacterium and also having a T-DNA chromosomal insertion ability that is lost or reduced in comparison to a wild-type Agrobacterium, thereby completing the present invention.

Thus, the present invention provides the following invention.
[1] An Agrobacterium, which has a T-DNA nuclear translocation ability equivalent to that of a wild-type Agrobacterium, and has a T-DNA chromosomal insertion ability that is lost or reduced in comparison to a wild-type Agrobacterium.
[2] The Agrobacterium according to [1], wherein homologous recombination efficiency upon its transformation in a plant is two or more times increased in comparison to a wild-type Agrobacterium, wherein the homologous recombination efficiency means the number of plants undergoing homologous recombination/the number of plants subjected to transformation.
[3] The Agrobacterium according to [1] or [2], wherein gene targeting efficiency upon its transformation in a plant is two or more times increased in comparison to a wild-type Agrobacterium, wherein the gene targeting efficiency means homologous recombination efficiency/random recombination efficiency, the homologous recombination efficiency means the number of plants undergoing homologous recombination/the number of plants subjected to transformation, and the random recombination efficiency means the number of plants undergoing random recombination/the number of plants subjected to transformation.
[4] The Agrobacterium according to any one of [1] to [3], wherein the gene targeting efficiency upon its transformation in a plant is 1% or more, wherein the gene targeting efficiency means homologous recombination efficiency/random recombination efficiency, the homologous recombination efficiency means the number of plants undergoing homologous recombination/the number of plants subjected to transformation, and the random recombination efficiency means the number of plants undergoing random recombination/the number of plants subjected to transformation.
[5] The Agrobacterium according to any one of [1] to [4], wherein the function of a gene associated with T-DNA random insertion is lost or reduced.
[6] The Agrobacterium according to [5], wherein the gene associated with T-DNA random insertion is one or more selected from among an ATU3081 gene, an ATU4309 gene, an ATU6150 (virH1 gene, an ATU6154 (virF) gene, an ATU6156 (virK) gene, an ATU6183 (virD3) gene, an ATU6184 (virD4) gene, an ATU6185 (virD5) gene, an ATU6188 (virE0) gene, an ATU6189 (virE1) gene, an ATU6191 (virE3) gene, an ATU6180 (virC1) gene, and a homologous gene thereof.
[7] The Agrobacterium according to any one of [1] to [6], which is Agrobacterium rhizogenes or Agrobacterium tumefaciens.
[8] The Agrobacterium according to any one of [1] to [7], which has a targeting vector comprising a foreign gene.
[9] A method for producing the Agrobacterium according to any one of [1] to [8], which comprises disrupting a gene associated with T-DNA random insertion in the chromosome of an Agrobacterium by a homologous recombination method.
[10] A plant cell which is obtained by infecting a plant cell with the Agrobacterium according to [8].
[11] A method for producing a transformed plant, which comprises infecting a plant cell with the Agrobacterium according to [8].

### Advantageous Effects of Invention

According to the present invention, gene targeting efficiency (homologous recombination efficiency) can be significantly increased by modifying an Agrobacterium gene that mediates transformation. According to the method of the present invention, by utilizing homologous recombination, a gene can be introduced with high efficiency into a specific site on the chromosome of a plant.

### Brief Description of Drawings

[Figure 1] Figure 1 shows confirmation of the gene disruption of an Agrobacterium by genomic PCR. W: a wild-type Agrobacterium strain, and M: an Agrobacterium gene disrupted strain.
[Figure 2] Figure 2 shows a gene targeting evaluation system in Arabidopsis thaliana.
[Figure 3] Figure 3 shows T1 seeds that emit GFP fluorescence.

### Embodiments for Carrying out the Invention

Hereinafter, the present invention will be described in detail.

The present invention relates to an Agrobacterium having a T-DNA nuclear translocation ability equivalent to that of a wild-type Agrobacterium and also having a T-DNA chromosomal insertion ability that is lost or reduced in comparison to a wild-type Agrobacterium. By transforming a plant with the Agrobacterium of the present invention, gene targeting efficiency can be increased.

Preferably, in the Agrobacterium of the present invention, the function of a gene associated with T-DNA random insertion is lost or reduced. The term "T-DNA random insertion" is used in the present description to include all types of T-DNA insertions, other than the insertion by homologous recombination. Specific examples of the gene associated with such T-DNA random insertion include, but are not limited to, a Vir gene, a ChvA gene, a ChvB gene, a PscA gene, an Att gene, and a function-unknown gene (unknown gene).

Examples of the transcription unit of the Vir gene include virA, virB, virC1, virD3, virD4, virD5, virE0, virE1, virE2, virF, virG, virH1, and virK. Each transcriptional region comprises one or plural genes. The Vir gene is preferably any one or more selected from among virA, virC1 virD3, virD4, virD5, virE0, virE1, virE2, virF, virH1 and virK, more preferably, any one or more selected from among virC1, virD3, virD4, virD5, virE0, virE1, virE2, virF, virH1 and virK, further preferably, any one or more selected from among virE0, virE1, virE2, virF, virH1 and virK, and particularly preferably, any one or more selected from among virE0, virE1 and virE2.

Specific examples of the Vir gene include an ATU6150 (virH1) gene, an ATU6154 (virF) gene, an ATU6166 (virA) gene, an ATU6183 (virD3) gene, an ATU6184 (virD4) gene, an ATU6185 (virD5) gene, an ATU6188 (virE0) gene, an ATU6189 (virE1) gene, an ATU6190 (virE2) gene, an ATU6191 (virE3) gene, an ATU6156 (virK) gene, and an ATU6180 (virC1) gene.

Among the above-described genes, the Vir gene is preferably any one or more selected from a TU6150 (virH1 gene, an ATU6154 (virF) gene, an ATU6183 (virD3) gene, an ATU6184 (virD4) gene, an ATU6185 (virD5) gene, an ATU6188 (virE0) gene, an ATU6189 (virE1) gene, an ATU6191 (virE3) gene, an ATU6156 (virK) gene, and an ATU6180 (virC1) gene.

The Vir gene is more preferably any one or more selected from an ATU6191 (virE3) gene, an ATU6150 (virH1 gene, an ATU6188 (virE0) gene, an ATU6154 (virF) gene, and an ATU6156 (virK) gene.

The Vir gene is further preferably any one or more selected from an ATU6191 (virE3) gene, an ATU6150 (virH1) gene, an ATU6154 (virF) gene, and an ATU6156 (virK) gene.

The Vir gene is particularly preferably any one or more selected from an ATU6191 (virE3) gene, an ATU6154 (virF) gene, and an ATU6156 (virK) gene.

The Vir gene is most preferably an ATU6191 (virE3) gene.

Examples of the function-unknown gene (unknown gene) include an ATU3081 gene and an ATU4309 gene.

The number of genes whose function is to be lost or reduced is not particularly limited, as long as it is 1 or greater, and may be any number of 1 to 11. For example, the functions of 1 to 10 genes, and preferably of 1 to 5 genes can be lost or reduced.

The nucleotide sequences of an ATU3081 gene (SEQ ID NO: 1), an ATU4309 gene (SEQ ID NO: 2), an ATU6150 (virH1) gene (SEQ ID NO: 3), an ATU6154 (virF) gene (SEQ ID NO: 4), an ATU6166 (virA) gene (SEQ ID NO: 5), an ATU6183 (virD3) gene (SEQ ID NO: 6), an ATU6184 (virD4) gene (SEQ ID NO: 7), an ATU6185 (virD5) gene (SEQ ID NO: 8), an ATU6188 (virE0) gene (SEQ ID NO: 9), an ATU6189 (virE1) gene (SEQ ID NO: 10), an ATU6190 (virE2) gene (SEQ ID NO: 11), an ATU6191 (virE3) gene (SEQ ID NO: 12), an ATU6156 (virK) gene (SEQ ID NO: 13) and an ATU6180 (virC1) gene (SEQ ID NO: 14) in the Agrobacterium A208 strain are shown in SEQ ID NOS: 1 to 14 in the sequence listing, respectively. The nucleotide sequences of anATU6150 (virH1) gene (SEQ ID NO: 15), an ATU6154 (virF) gene (SEQ ID NO: 16), an ATU6166 (virA) gene (SEQ ID NO: 17), an ATU6183 (virD3) gene (SEQ ID NO: 18), an ATU6184 (virD4) gene (SEQ ID NO: 19), an ATU6185 (virD5) gene (SEQ ID NO: 20), an ATU6189 (virE1) gene (SEQ ID NO: 21), an ATU6190 (virE2) gene (SEQ ID NO: 22), an ATU6191 (virE3) gene (SEQ ID NO: 23), an ATU6156 (virK) gene (SEQ ID NO: 24) and an ATU6180 (virC1) gene (SEQ ID NO: 25) in the Agrobacterium EHA101 strain are shown in SEQ ID NOS: 15 to 25 in the sequence listing, respectively.

The Agrobacterium of the present invention does not only include an Agrobacterium, in which the functions of the aforementioned 14 types of genes are lost or reduced, but it also includes an Agrobacterium, in which the functions of homologous genes of the aforementioned genes are lost or reduced. The homologous gene means a gene, which consists of a nucleotide sequence having homology (which is also referred to as "sequence identity") of 40% or more, preferably 50% or more, more preferably 60% or more, even more preferably 65% or more, further preferably 70% or more, further preferably 75% or more, further preferably 80% or more, still further preferably 85% or more, still further preferably 90% or more, and still further preferably 95% or more, with the nucleotide sequence of any one of an ATU3081 gene, anATU4309 gene, an ATU6150 (virH1) gene, an ATU6154 (virF) gene, an ATU6166 (virA) gene, an ATU6183 (virD3) gene, an ATU6184 (virD4) gene, an ATU6185 (virD5) gene, an ATU6188 (virE0) gene, an ATU6189 (virE1) gene, an ATU6190 (virE2) gene, an ATU6191 (virE3) gene, an ATU6156 (virK) gene, and an ATU6180 (virC1) gene, and which has functions equivalent to those of each of the above-described genes. The following Table 1 shows the gene sizes and homology levels of homologous genes in the Agrobacterium EHA101 strain, as several examples of the homologous genes.

[Table 1]

**Table 1:**

| Gene name | Accession | Gene size (Agrobacterium A208 strain) | Gene size (Agrobacterium EHA101 strain) | Homology (DNA level) |
|---|---|---|---|---|
| - | ATU3081 | 1494 bp | 1494 bp | 100% |
| - | ATU4309 | 1032 bp | 1032 bp | 100% |
| virH1 | ATU6150 | 1260 bp | 1269 bp | 79% |
| virF | ATU6154 | 939 bp | 942 bp | 45% |
| virA | ATU6166 | 2502 bp | 2498 bp | 76% |
| virD3 | ATU6183 | 2022 bp | 639 bp | 49% |
| virD4 | ATU6184 | 2007 bp | 1959 bp | 78% |
| virD5 | ATU6185 | 2523 bp | 2511 bp | 69% |
| virE0 | ATU6188 | 252 bp | - | - |
| virE1 | ATU6189 | 192 bp | 198 bp | 70% |
| virE2 | ATU6190 | 1671 bp | 1650 bp | 72% |
| virE3 | ATU6191 | 2055 bp | 2019 bp | 69% |
| virK | ATU6156 | 435bp | 438bp | 75% |
| virC1 | ATU6180 | 696bp | 696bp | 81% |

The method of losing or reducing the functions of the above described genes is not particularly limited. A mutation or deletion may be introduced into the nucleotide sequences of the above-described one or more genes, so as to lose or reduce the functions of the gene(s), or all of the above-described one or more genes may be deleted, so as to delete the functions of the gene(s). Methods of site-specifically mutating a gene on the chromosome are well known to a person skilled in the art. Representative examples of such a method include a method of utilizing the mechanism of transposon and homologous recombination (Ohman et al., J. Bacteriol., 162: 1068-1074 (1985)) and a method involving, as principles, site-specific integration caused by the mechanism of homologous recombination and dropping caused by homologous recombination as a second stage (Noti et al., Methods Enzymol., 154: 197-217 (1987)). Moreover, there can also be utilized a method which comprises allowing a sacB gene derived from Bacillus subtilis to coexist with a concerned gene, and then easily isolating, as a sucrose resistance strain, a microorganism strain in which the concerned gene has been dropped by the second-stage homologous recombination (Schweizer, Mol. Microbiol., 6: 1195-1204 (1992), Lenz et al., J. Bacteriol., 176: 4385-4393 (1994)). However, the method is not particularly limited. Preferably, a gene associated with T-DNA random insertion, which is in the chromosome of an Agrobacterium, can be disrupted according to a homologous recombination method.

In addition, the present invention relates to a method for producing a transformed plant, which comprises infecting plant cells with an Agrobacterium having a T-DNA nuclear translocation ability equivalent to that of a wild-type Agrobacterium and also having a T-DNA chromosomal insertion ability that is lost or reduced in comparison to a wild-type Agrobacterium, wherein the Agrobacterium has a targeting vector comprising a foreign gene.

The "plant transformation method that involves the mediation of Agrobacterium," which becomes a base of the method of the present invention, is generally referred to as an "Agrobacterium-mediated transformation method" or an "Agrobacterium method." This is a method of transforming a plant by introducing a foreign gene into the genome of a plant cell through the mediation of Agrobacterium. Such Agrobacterium inserts a T-DNA region contained in a vector, such as a plasmid, possessed by the Agrobacterium itself, into the plant chromosomal DNA, in the plant cells infected with the Agrobacterium. Thus, according to the plant transformation method involving the mediation of Agrobacterium, under the control of a promoter and a terminator present between the right border sequence (RB) and the left border sequence (LB) of a T-DNA region, a vector (preferably, a binary vector), into which a foreign gene to be introduced into a plant has been incorporated, is introduced into Agrobacterium according to an ordinary method, and then, the Agrobacterium is inoculated into the plant, and the plant is thereby infected therewith, so that the foreign gene in the T-DNA region can be introduced into the plant.

More specifically, for example, in the method of the present invention, a transformation method, which comprises infecting the callus or tissue section of a plant in an in vitro culture system with Agrobacterium, so that a foreign gene is introduced therein, and then regenerating the foreign gene in the plant body by in vitro culture, so as to produce a transformed plant body, may be used as a plant transformation method involving the mediation of Agrobacterium. Alternatively, in the method of the present invention, an in planta transformation method, which is generally used for Arabidopsis thaliana and is called "Floral dip method," may also be used. Furthermore, in the method of the present invention, the in planta transformation method comprising inoculating a foreign gene-containing vector into Agrobacterium and then infecting the meristem of an individual plant (a plant body or a seed) with the Agrobacterium can be preferably used as a plant transformation method involving the mediation of Agrobacterium.

In one embodiment, in the method of the present invention, a plant can also be transformed according to a plant transformation method involving the mediation of Agrobacterium, particularly, using Agrobacterium retaining a vector having a T-DNA region that contains a foreign gene but does not contain a selection marker gene, namely, a vector into the T-DNA region of which a foreign gene has been introduced (a foreign gene-containing vector) but a selection marker gene has not been inserted therein. The "foreign gene" in the T-DNA region used herein is not a selection marker gene. The definition, specific examples and the like of the term "selection marker gene" will be described later. In this method of the present invention, a selection marker gene usually used for selection of a transformant is not introduced, together with a foreign gene, into host plant cells, and a foreign gene introduced into the genome, or a nucleic acid such as mRNA or a protein expressed from the gene, is detected, so that a plant transformant may be selected. Since this plant transformant does not contain a selection marker gene, it cannot be selected by a selection method using a selection marker (for example, selection based on antibiotic resistance or herbicide resistance). In this method, by using the method in combination with an Agrobacterium transformation method having high transformation efficiency (for example, the after-mentioned method), a transformant can be efficiently obtained without introducing a selection marker gene into the genome of a host plant.

In the present invention, by using an Agrobacterium strain in which a specific gene is disrupted, homologous recombination efficiency can be increased. This is considered because insertion of T-DNA into plant genome is suppressed by deletion of a specific gene, and as a result, homologous recombination is promoted.

A bacterial suspension of Agrobacterium to be inoculated into a plant may further comprise a surfactant such as Tween 20. Such a bacterial suspension of Agrobacterium may also comprise phenols such as acetosyringone. These components are able to further improve transformation efficiency.

The plant transformation method of the present invention can be applied to any given plants that can be infected with Agrobacterium. The target, to which the plant transformation method of the present invention can be applied, may be either a dicotyledon or a monocotyledon. The target plant, to which the present plant transformation method can be applied, is not particularly limited. Examples of the target plant include plants belonging to: Poaceae [wheat (Triticum aestivum L.), rice (Oryza sativa), barley (Hordeum vulgare L.), com (Zea mays L.), sorghum (Sorghum bicolor (L.) Moench), erianthus (Erianthus spp), Guinea grass (Panicum maximum Jacq.), miscanthus (Miscanthus spp), sugarcane (Saccharum officinarum L.), napier grass (Pennisetum purpureum Schumach), pampas grass (Cortaderia argentea Stapf.), Perennial ryegrass (Lolium perenne L.), Italian ryegrass (Lolium multiflorum Lam.), meadow fescue (Festuca pratensis Huds.), tall fescue (Festuca arundinacea Schreb.), Orchard grass (Dactylis glomerata L.), timothy (Phleum pratense L.), etc.]; Leguminosae [soybean (Glycine max), adzuki bean (Vigna angularis Willd.), kidney bean (Phaseolus vulgaris L.), fava bean (Vicia faba L.), etc.]; Malvaceae [cotton (Gossypium spp.), kenaf (Hibiscus cannabinus), okra (Abelmoschus esculentus), etc.]; Solanaceae [eggplant (Solanum melongena L.), tomato (Solanum lycopersicum), green peper (Capsicum annuum L. var. angulosum Mill.), capsicum (Capsicum annuum L.), tobacco (Nicotiana tabacum L.), etc.]; Brassicaceae [Arabidopsis (Arabidopsis thaliana), rape (Brassica campestris L.), Chinese cabbage (Brassica pekinensis Rupr.), cabbage (Brassica oleracea L. var. capitata L.), radish (Raphanus sativus L.), rape seed (Brassica campestris L., B. napus L.), etc.]; Cucurbitaceae [cucumber (Cucumis sativus L.), melon (Cucumis melo L.), watermelon (Citrullus vulgaris Schrad.), pumpkin (C. moschata Duch., C. maxima Duch.), etc.]; Convolvulaceae [sweet potato (Ipomoea batatas), etc.], Liliaceae [spring onion (Allium fistulosum L.), onion (Allium cepa L.), Chinese chive (Allium tuberosum Rottl.), garlic (Allium sativum L.), asparagus (Asparagus officinalis L.), etc.]; Labiatae [Japanese basil (Perilla frutescens Britt. var. crispa), etc.]; Asteraceae [chrysanthemum (Chrysanthemum morifolium), garland chrysanthemum (Chrysanthemum coronarium L.), lettuce (Lactuca sativa L. var. capitata L.), etc.]; Rosaceae [rose (Rose hybrida Hort.), strawberry (Fragaria x ananassa Duch.), etc.]; Rutaceae [orange (Citras unshiu), Japanese pepper (Zanthoxylum piperitum DC.), etc.]; Myrtaceae [eucalyptus (Eucalyptus globulus Labill), etc.], Salicaceae [poplar (Populas nigra L. var. italic Koehne), etc.]; Chenopodiaceae [spinach (Spinacia oleracea L.), sugar beet (Beta vulgaris L.), etc.]; Gentianaceae [gentian (Gentiana scabra Bunge var. buergeri Maxim.), etc.]; and Caryophyllaceae [carnation (Dianthus caryophyllus L.), etc.]. Plants, which have provided only low gene transfer efficiency by the conventional transformation methods, such as poaceous, leguminous and malvaceous plants, are particularly preferable as target plants, to which, the transformation method of the present invention is applied.

The Agrobacterium used in the method of the present invention is not particularly limited, as long as it is a plant pathogen belonging to the genus Rhizobium, capable of causing transformation mediated by Agrobacterium. Examples of such Agrobacterium include Agrobacterium tumefaciens, Agrobacterium vitis, Agrobacterium rhizogenes, and Agrobacterium radiobacter. Specific examples of the Agrobacterium include, but are not limited to, Agrobacterium tumefaciens LBA4404 strain, C58 strain, EHA101 strain, A208 strain, Agrobacterium vitis F2/5 strain, S4 strain, and Agrobacterium rhizogenes A4 strain, LBA9402 strain, and the derived strains thereof.

A foreign gene to be introduced into a plant is introduced into a T-DNA region of a vector comprising the T-DNA region to produce a foreign gene-containing vector (i.e., a targeting vector having a foreign gene), and the thus produced vector is then introduced into Agrobacterium, thereby producing an Agrobacterium having a foreign gene-containing vector. The vector comprising the T-DNA region comprises a T-DNA region derived from an Agrobacterium plasmid, namely, a nucleic acid sequence sandwiched between a right border sequence (RB) and a left border sequence (LB), and a replication origin, and this is a vector autonomously replicating in Agrobacterium. The T-DNA region in the vector preferably comprises a promoter and a terminator between the RB and LB sequences. The vector comprising the T-DNA region is more preferably a binary vecor, which comprises the repication origins of other microorganisms such as E. coli or yeast, and is capable of autonomously replicating also in those microorganisms. The vector comprising the T-DNA region may comprise a vir gene outside of the T-DNA region. As vectors comprising a T-DNA region for plant transformation, which are preferably used for introduction of a foreign gene into a plant, multiple types of vectors are commerially available. Examples of the vector comprising the T-DNA region include, but are not limited to, pIG121-Hm (Ohta, S., et al., Plant Cell Physiol. 31, 805-813. 1990), pCAMBIA (Marker Gene Technologies, Inc.), pRI909 (TaKaRa), pRI101 (TaKaRa), pBI (Inplanta Innovations Inc.), pBIN (Bevan, M. et al., Nucleic Acids Res. 12, 8711-8721. 1984), pPZP (Hajdukiewicz, P. et al., Plant Mol. Biol. 25, 989-994 1994), pGreen (Hellens, R. et al., Plant Mol. Biol. 42, 819-832. 2000), pBIBAC (Liu, Y. et al., Proc. Natl. Acad. Sci. USA 96, 6535-6540. 1999), pGA, SEV, pEND4K, pCIB10, pMRK63, pGPTV, pCGN1547, pART, pGKB5, pMJD80, pMJD81, pBINPLUS, pRT100, pCB, pMDC, pRCS2, and pORE.

In a preferred embodiment of the present invention, a vector into the T-DNA region of which a foreign gene has been introduced (a foreign gene-containing vector) may comprise a selection marker gene outside of the T-DNA region. However, it is preferable that such a selection marker gene be not comprised in the T-DNA region. The selection marker gene is used in the present invention to mean a gene imparting survival ability only to transformed cells under predetermined conditions, and enabling selection of the transformed cells, or a fluorescent protein gene or an enzyme gene catalyzing a coloring reaction. Representative examples of the selection marker gene include a drug resistance gene and a nutrient-requiring complementary gene. Specific examples of the selection marker gene include, but are not limited to, a kanamycin resistance gene, a hygromycin resistance gene, a phosphinothricin resistance gene, a bialaphos resistance gene, a gentamicin resistance gene, a sulfonylurea resistance gene, a dihydrofolate reductase gene, a bleomycin resistance gene, a luciferase gene, a β-galactosidase gene, a β-glucuronidase gene, and a green fluorescence protein (GFP) gene. Since the foreign gene-containing vector does not comprise a selection marker gene in the T-DNA region thereof, the selection marker gene is not introduced into a transformed plant, so that a transformed plant with higher trait stability and higher safety can be obtained. Since transformation efficiency is significantly increased in the method of the present invention, a transformed plant can be selected with sufficient efficiency, without introduction of a selection marker gene, and thus, without using a change in the phenotype caused by the expression of the selection marker gene as an indicator.

A foreign gene to be comprised in a vector comprising a T-DNA region is any given gene intended to induce expression in a plant, and it may be either a plant-derived gene or an animal-derived gene. The "foreign gene" indicates a nucleic acid (generally, DNA) introduced from the outside through the mediation of Agrobacterium, and it may be, for example, a gene that may be isolated from a host plant into which the gene is to be introduced, or from an organism species or strain of the same species as the host plant. The "foreign gene" of the present invention may encode a protein or may also encode functional RNA. Examples of the foreign gene include a group of genes, which are associated with seed yield increase, environmental stress (low temperature, drying, salts, virus, disease, and high temperature) resistance, photosynthetic function enhancement, biomass production, useful substance production, etc., but examples are not limited thereto.

Introduction of a foreign gene-containing vector into Agrobacterium can be carried out according to an ordinary method. For example, a foreign gene-containing vector can be introduced into Agrobacterium according to a freezing-thawing method or a particle gun method. To explain an example of the freezing-thawing method just briefly, competent cells of Agrobacterium are mixed with a foreign gene-containing vector, the obtained mixture is then incubated on ice for 5 minutes, and it is then frozen in liquid nitrogen for 5 minutes. Subsequently, the resultant is incubated at 37°C for 5 minutes, so that it is thawed, and it is then subjected to a shaking culture at room temperature or at 28°C for 2 to 4 hours. Thereafter, the reaction culture is cultured in an antibiotic-containing medium, and the formed single clones may be collected.

In the present invention, plant cells can be infected with an Agrobacterium. A transformed plant can be produced by infecting plant cells with an Agrobacterium having a foreign gene-containing vector.

Inoculation of Agrobacterium may be carried out according to an ordinary method. In the present invention, in the case of using an in planta transformation method, an Agrobacterium having a foreign gene-containing vector is preferably inoculated into the meristem of an individual plant (a plant body or a seed). Any given meristem of an individual plant can be used as an inoculation site. Such an Agrobacterium is preferably inoculated into, for example, the meristem of the shoot apex or axillary bud of a juvenile plant or seedling, or the meristem of the embryo of a seed. In the case of a poaceous plant for example, an Agrobacterium is preferably inoculated into the meristem of the embryo of a seed. Inoculation of an Agrobacterium into the meristem of such a seed embryo may be inoculation of an Agrobacterium into a shoot (stem and leave) germinating from a seed, an embryonic portion around a shoot base portion, or a root.

Inoculation of Agrobacterium into the meristem is preferably carried out in a wound site on the meristem. Such a wound site may be created, for example, by pricking several sites on the meristem with a sterilized needle (for example, with a diameter of 0.71 mm) to make stab wounds. The size of such stab wounds may be approximately 0.5 mm to 2 mm, but it is not limited thereto. Otherwise, other types of wounds, such as small incised wounds, may also be created on the meristem. After creation of wounds, Agrobacterium may be inoculated into such wound sites, or after inoculation of Agrobacterium into a plant, wounds may be created on the inoculation sites.

In order to promote infection with Agrobacterium, a plant, into which such Agrobacterium has been inoculated, may be co-cultured with Agrobacterium. In the method of the present invention, the co-culture is preferably carried out at a temperature of 23°C or higher, preferably at 25°C to 30°C, and more preferably at 28°C. The co-culture may be carried out for an ordinary period of time. For example, the co-culture is preferably carried out, for example, for 12 hours to 10 days, preferably for 24 hours, to 5 days, and more preferably for 36 hours to 4 days. By performing a co-culture in such a temperature range, the growth of Agrobacterium can be promoted, and thus, infection can also be promoted.

After completion of the infection, a plant into which Agrobacterium has been inoculated may be subjected to a treatment of eliminating the Agrobacterium. The Agrobacterium elimination treatment can be carried out by treating the Agrobacterium with an antibiotic such as cefotaxime.

A plant, which has been infected with Agrobacterium and then, as necessary, has been disinfected, is allowed to grow under suitable cultivation conditions. At a time point in which the plant has reached a predetermined growing stage, it is preferably confirmed by genomic PCR or the like that the introduced foreign gene has been incorporated into the genome. The plant, in which introduction of the foreign gene has been confirmed, is selected as a transformed plant, and this plant is defined as T0 generation.

The plant of T0 generation is allowed to form a flower bud, and is then allowed to bleed, so that the plant forms seeds. The thus obtained plant is a plant of T1 generation. Even in the case of the plant of T1 generation, a stable transformed plant can be selected and obtained by confirming incorporation of a foreign gene into the genome.

With regard to the thus obtained transformed plant, gene targeting efficiency can be calculated. Such gene targeting efficiency can be calculated according to the expression: homologous recombination efficiency/random recombination efficiency. Herein, the term "homologous recombination efficiency" is used to mean the number of plants undergoing homologous recombination/the number of plants subjected to transformation, whereas the term "random recombination efficiency," is used to mean the number of plants undergoing random recombination/the number of plants subjected to transformation.

The homologous recombination efficiency obtained upon transformation of a plant with the Agrobacterium of the present invention is increased, preferably 2 time or more, more preferably 5 time or more, even more preferably 10 time or more, further preferably 15 time or more, and particularly preferably 20 time or more, in comparison to that of a wild-type Agrobacterium. The higher the aforementioned improvement magnification, the more preferable it is. The upper limit is not particularly limited, and it is generally 200 time or less, and for example, 100 time or less.

The gene targeting efficiency obtained upon transformation of a plant with the Agrobacterium of the present invention is increased, preferably 2 time or more, more preferably 4 time or more, even more preferably 10 time or more, further preferably 15 time or more, and particularly preferably 20 time or more, in comparison to that of a wild-type Agrobacterium. The higher the aforementioned improvement magnification, the more preferable it is. The upper limit is not particularly limited, and it is generally 200 time or less, and for example, 100 time or less.

The gene targeting efficiency obtained upon transformation of a plant with the Agrobacterium of the present invention is preferably 1% or more, more preferably 1.5% or more, even more preferably 5% or more, further preferably 8% or more, and particularly preferably 10% or more. The higher the gene targeting efficiency, the more preferable it is. The upper limit is not particularly limited, and it is generally 70% or less, and for example, 50% or less.

### Examples

Hereinafter, the present invention will be more specifically described in the following examples. However, these examples are not intended to limit the technical scope of the present invention.

### [Example 1] Production of gene disrupted strain of Agrobacterium

Gene disrupted strains of Agrobacterium A208 (C58 chromosome, nopaline-type T37pTi) (Wirawan IG, Kang HW, Kojima M (1993) Isolation and characterization of a new chromosomal virulence gene of Agrobacterium tumefaciens. J Bacteriol 175: 3208-3212) were produced by applying a "method comprising, as principles, site-specific incorporation caused by the mechanism of homologous recombination and dropping by homologous recombination as a second stage," which is described in JP Patent Publication (Kokai) No. 2007-259708 A.

Primers used for production of gene disrupted strains of Agrobacterium will be shown below.

| | |
|---|---|
| ATU3081 Forward1 | TTAGCGCCTCTGAAATTCGG (SEQ ID NO: 26) |
| ATU3081 Reverse1 | AAGGCACTTCGTCAGCGACCA (SEQ ID NO: 27) |
| ATU3081 Forward2 | TTACTTCTGACCGCAAGAGATC (SEQ ID NO: 28) |
| ATU3081 Reverse2 | TTCGCCAGATGCTCAGCGAT (SEQ ID NO: 29) |

| | |
|---|---|
| ATU4309 Forward1 | GGTGGTTGGAAAGCTCATGTC (SEQ ID NO: 30) |
| ATU4309 Reverse1 | CGATCCAGAAAATCCTGGCG (SEQ ID NO: 31) |
| ATU4309 Forward2 | CACCGGCTTCTGCGACGCGC (SEQ ID NO: 32) |
| ATU4309 Reverse2 | AATCTATCCGTAGATCATCCTC (SEQ ID NO: 33) |

| | |
|---|---|
| ATU6150 Forward1 | GTGGATATCGAGGGGACAC (SEQ ID NO: 34) |
| ATU6150 Reverse1 | ACATACTTTCATCGAAGTCG (SEQ ID NO: 35) |
| ATU6150 Forward2 | GGTCACCGTTCCGTTCGGTT (SEQ ID NO: 36) |
| ATU6150 Reverse2 | TCTCCGCGAACAGATCTACTG (SEQ ID NO: 37) |

| | |
|---|---|
| ATU6154 Forward1 | CAATTGACTGACCTCGCGAA (SEQ ID NO: 38) |
| ATU6154 Reverse1 | CGTGCATGCTCCTTCTTTCT (SEQ ID NO: 39) |
| ATU6154 Forward2 | TTCCTGGATCCACCGCGCTG (SEQ ID NO: 40) |
| ATU6154 Reverse2 | ATCGGCTCAATTGATATCCC (SEQ ID NO: 41) |

| | |
|---|---|
| ATU6166 Forward1 | TTTGCTTGGTCACCTGAAAC (SEQ ID NO: 42) |
| ATU6166 Reverse1 | CCGCACTTACGTCCTCGTAC (SEQ ID NO: 43) |
| ATU6166 Forward2 | AGTTGAGGArGTTTTTCAGGAG (SEQ ID NO: 44) |
| ATU6166 Reverse2 | TAGGGTCGGTTACTCTAGGAT (SEQ ID NO: 45) |

| | |
|---|---|
| ATU6183 Forward1 | AAACCGCACTCCCGATATTC (SEQ ID NO: 46) |
| ATU6183 Reverse1 | CACTCGGTCCTTCTCTATCT (SEQ ID NO: 47) |
| ATU6183 Forward2 | AGGCTGTGTTGTTCGCAGCA (SEQ ID NO: 48) |
| ATU6183 Reverse2 | CCAAAGATTGGCCCCTTGATAC (SEQ ID NO: 49) |

| | |
|---|---|
| ATU6184 Forward1 | GTTGGTTCCCAATGCCAATC (SEQ ID NO: 50) |
| ATU6184 Reverse1 | CACTTCACCGAGATTCTTCG (SEQ ID NO: 51) |
| ATU6184 Forward2 | CTTCAAGCTGCCTTTCACAT (SEQ ID NO: 52) |
| ATU6184 Reverse2 | AAGATCAAAGGCGACTCCTC (SEQ ID NO: 53) |

| | |
|---|---|
| ATU6185 Forward1 | GCCTTTCACATTGGAATCAT (SEQ ID NO: 54) |
| ATU6185 Reverse1 | ATGGGAAGACCTCGTTGTCA (SEQ ID NO: 55) |
| ATU6185 Forward2 | AAGGTCGTCCACGATACTTT (SEQ ID NO: 56) |
| ATU6185 Reverse2 | GGCACTGCTGTCAAGAAATC (SEQ ID NO: 57) |

| | |
|---|---|
| ATU6188 Forward1 | ATTCCTGAGCATTGAGGTCC (SEQ ID NO: 58) |
| ATU6188 Reverse1 | TGCTTTCTAGGCTGCTGCAG (SEQ ID NO: 59) |
| ATU6188 Forward2 | ATGGAGCAAACCTTAATTTG (SEQ ID NO: 60) |
| ATU6188 Reverse2 | GGACATCCCGGTGGAATTTA (SEQ ID NO: 61) |

| | |
|---|---|
| ATU6189 Forward1 | ACCGAAGGCTCAATTCTATT (SEQ ID NO: 62) |
| ATU6189 Reverse1 | ATCATTGTTTCTCCTACAGA (SEQ ID NO: 63) |
| ATU6189 Forward2 | AAGGAGTTAGACGATGGATC (SEQ ID NO: 64) |
| ATU6189 Reverse2 | CAGCTCGCATTGAATTCCG (SEQ ID NO: 65) |

| | |
|---|---|
| ATU6190 Forward1 | ACCTGGCGGCAAACCGACA (SEQ ID NO: 66) |
| ATU6190 Reverse1 | TAGCCGGCTAGGTTTTCTTC (SEQ ID NO: 67) |
| ATU6190 Forward2 | GGTCGACACGACGAAGAAG (SEQ ID NO: 68) |
| ATU6190 Reverse2 | TATITCACATGCTTCGCGCG (SEQ ID NO: 69) |

| | |
|---|---|
| ATU6191 Forward1 | ACCGCGTCAGTGACGAAGCC (SEQ ID NO: 70) |
| ATU6191 Reverse1 | GCGTTACGCTTCAGAACCTT (SEQ ID NO: 71) |
| ATU6191 Forward2 | TGAAACAGGCCAAGATGTG (SEQ ID NO: 72) |
| ATU6191 Reverse2 | TCATTCACCATCGCGCGCCA (SEQ ID NO: 73) |

| | |
|---|---|
| ATU6156 Forward1 | ATCAATTGAGCCGATCTTCC (SEQ ID NO: 74) |
| ATU6156 Reverse1 | AGGTGATCCTATTTAGATTG (SEQ ID NO: 75) |
| ATU6156 Forward2 | TGATCTCTGACTCCTGTGT (SEQ ID NO: 76) |
| ATU6156 Reverse2 | CTTCCATGTGCTGATITCCA (SEQ ID NO: 77) |

| | |
|---|---|
| Atu6180 Forward1 | TCTCTGCGTCGATTTCAAGA (SEQ ID NO: 78) |
| Atu6180 Reverse1 | TCCTTATCCTGTCGATTTTG (SEQ ID NO: 79) |
| Atu6180 Forward2 | CAGATGGGAATTCGCAAACC (SEQ ID NO: 80) |
| Atu6180 Reverse2 | TGTGTGAGGGATCGGATAAC (SEQ ID NO: 81) |

Moreover, gene disruption was confirmed by performing genomic PCR on the produced Agrobacterium strains. Conditions for the PCR reaction are shown in the following Table 2.

[Table 2]

**Table 2:**

| Step | °C | Time | Number of cycles |
|---|---|---|---|
| 1 | 94 | 3 min | 1 |
| 2 | 94 | 30 sec | 35 |
| 3 | 57 | 30 sec | |
| 4 | 72 | 3 min | |
| 5 | 72 | 5 min | 1 |
| 6 | 4 | ∞ | |

In the genomic PCR performed to confirm gene disruption, the following primers were used.

| | |
|---|---|
| ATU3081 Forward1 | TTAGCGCCTCTGAAATTCGG (SEQ ID NO: 26) |
| ATU3081 Reverse2 | TTCGCCAGATGCTCAGCGAT (SEQ ID NO: 29) |
| ATU4309 Forward1 | GGTGGTTGGAAAGCTCATGTC (SEQ ID NO: 30) |
| ATU4309 Reverse2 | AATCTATCCGTAGATCATCCTC (SEQ ID NO: 33) |
| ATU6150 Forward1 | GTGGATATCGAGGGGACAC (SEQ NO: 34) |
| ATU6150 Reverse2 | TCTCCGCGAACAGATCTACTG (SEQ ID NO: 37) |
| ATU6154 Forward1 | CAATTGACTGACCTCGCGAA (SEQ ID NO: 38) |
| ATU6154 Reverse2 | ATCGGCTCAATTGATATCCC (SEQ ID NO: 41) |
| ATU6166 Forward1 | TTTGCTTGGTCACCTGAAAC (SEQ ID NO: 42) |
| ATU6166 Reverse2 | TAGGGTCGGTTACTCTAGGAT (SEQ ID NO: 45) |
| ATU6183 Forward1 | AAACCGCACTCCCGATATTC (SEQ ID NO: 46) |
| ATU6183 Reverse2 | CCAAAGATTGGCCCCTTGATAC (SEQ NO: 49) |
| ATU6184 Forward1 | GTTGGTTCCCAATGCCAATC (SEQ ID NO: 50) |
| ATU6184 Reverse2 | AAGATCAAAGGCGACTCCTC (SEQ ID NO: 53) |
| ATU6185 Forward1 | GCCTTTCACATTGGAATCAT (SEQ ID NO: 54) |
| ATU6185 Reverse2 | GGCACTGCTGTCAAGAAATC (SEQ ID NO: 57) |
| ATU6188 Forward1 | ATTCCTGAGCATTGAGGTCC (SEQ ID NO: 58) |
| ATU6188 Reverse2 | GGACATCCCGGTGGAATTTA (SEQ ID NO: 61) |
| ATU6189 Forward1 | ACCGAAGGCTCAATTCTATT (SEQ ID NO: 62) |
| ATU6189 Reverse2 | CAGCTCGCATTGAATTCCG (SEQ ID NO: 65) |
| ATU6190 Forward1 | ACCTGGCGGCAAACCGACA (SEQ ID NO: 66) |
| ATU6190 Reverse2 | TATTTCACATGCTTCGCGCG (SEQ ID NO: 69) |
| ATU6191 Forward1 | ACCGCGTCAGTGACGAAGCC (SEQ ID NO: 70) |
| ATU6191 Reverse2 | TCATTCACCATCGCGCGCCA (SEQ ID NO: 73) |
| ATU6156 Forward1 | ATCAATTGAGCCGATCTTCC (SEQ ID NO: 74) |
| ATU6156 Reverse2 | CTTCCATGTGCTGATTTCCA (SEQ ID NO: 77) |
| Atu6180 Forward1 | TCTCTGCGTCGATTTCAAGA (SEQ ID NO: 78) |
| Atu6180 Reverse2 | TGTGTGAGGGATCGGATAAC (SEQ ID NO: 81) |

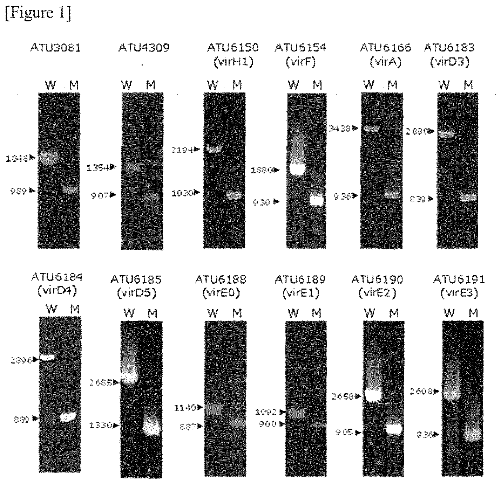

As a result of the genomic PCR, it was confirmed that a gene of interest was disrupted in each of the Agrobacterium gene disrupted strains (Figure 1). In addition, a sequencing analysis was carried out on each disrupted strain, and it was confirmed that the gene was disrupted at a DNA sequence level.

### [Example 2] Confirmation of pathogenicity (T-DNA chromosomal insertion ability) and T-DNA nuclear translocation ability of Agrobacterium gene disrupted strains

The pathogenicity (T-DNA chromosomal insertion ability) of the Agrobacterium gene disrupted strains was confirmed by the following method. The Agrobacterium gene disrupted strains produced in [Example 1] were each cultured in an LB solid medium (Difco) at 25°C for 48 hours, and the cultured cells were then inoculated into a leaf of a plant body of kalanchoe (Kalanchoe daigremontiana). The presence or absence of pathogenicity (T-DNA chromosomal insertion ability) was determined three weeks after the inoculation, based on the presence or absence of formation of a tumor (crown gall) in the inoculation site. Moreover, the size of the tumor was compared with that of a wild-type Agrobacterium strain, and the strength of pathogenicity was evaluated.

Moreover, T-DNA nuclear translocation ability was confirmed based on the transient expression of a GUS gene. The method will be described below. Strains were prepared by introducing a plasmid vector pIG121-Hm into the Agrobacterium gene disrupted strains produced in [Example 1], and they were then cultured in an LB solid medium (Difco) at 25°C for 48 hours. The cultured cells were then inoculated into a leaf of a plant body of kalanchoe (Kalanchoe daigremontiana). Two weeks after the inoculation, GUS activity was measured. Such GUS activity was measured by the method described in Journal of Bioscience and Bioengineering Vol. 90, 328-331. 2000.

The measurement results of the pathogenicity (T-DNA chromosomal insertion ability) and T-DNA nuclear translocation ability of the Agrobacterium gene disrupted strains are shown in Table 3. Evaluation standards of pathogenicity (T-DNA chromosomal insertion ability) and T-DNA nuclear translocation ability are as follows.

### Pathogenicity (T-DNA chromosomal insertion ability):

++: Tumor size is equivalent to that of the wild type (A208) (size: 0.5 times or greater) (T-DNA chromosomal insertion ability is "strong")
+: Tumor size is less than half of the wild type (A208) (T-DNA chromosomal insertion ability is "weak")
-: Tumor cannot be confirmed at all by visual observation (T-DNA chromosomal insertion ability "lacked")

### T-DNA nuclear translocation ability:

++: with T-DNA nuclear translocation ability (GUS activity value is 100 [pmol of 4-MU/min/mg protein] or more)
+: with T-DNA nuclear translocation ability (GUS activity value is 20 to 100 [pmol of 4-MU/min/mg protein])
-: without T-DNA nuclear translocation ability (GUS activity value is 20 [pmol of 4-MU/min/mg protein] or less)

[Table 3]

**Table 3:**

| Agrobacterium endogenous gene to be disrupted | T-DNA nuclear translocation ability | T-DNA chromosomal insertion ability |
|---|---|---|
| Agrobacterium wild-type strain (control) | ++ | ++ |
| ATU3081 | ++ | - |
| ATU6154 (virF) | ++ | + |
| ATU6156 (virK) | ++ | - |
| ATU6166 (virA) | + | - |
| ATU6185 (virD5) | ++ | - |
| ATU6190 (virE2) | + | - |
| ATU6184 (virD4) | + | - |
| ATU6189 (virE1) | ++ | - |
| ATU6191 (virE3) | ++ | + |
| ATU6150 (virH1) | ++ | + |
| ATU6180 (virC1) | ++ | + |
| ATU6183 (virD3) | ++ | + |
| ATU6188 (virE0) | ++ | + |
| ATU4309 | ++ | + |

### [Example 3] Construction of gene targeting (homologous recombination) evaluation system using Arabidopsis thaliana

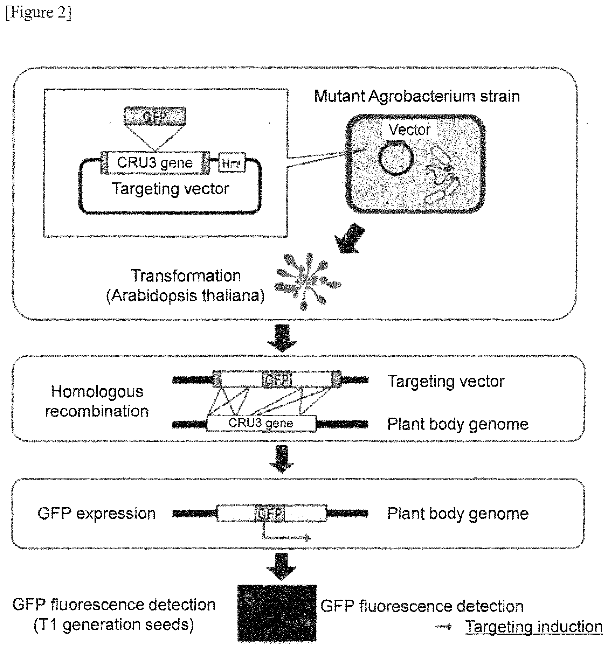

Cruciferin 3 (hereinafter referred to as "CRU3") that is a seed storage protein of Arabidopsis thaliana was used to produce a targeting reporter gene, and a gene targeting evaluation system was then constructed in Arabidopsis thaliana which is a dicotyledon (Figure 2). Since a GFP fluorescence protein (GFP) has been inserted into a CRU3 gene comprised in a targeting vector to be used, when gene targeting is successfully carried out, specific GFP fluorescence is considered to be emitted in the endosperm that is an expression site of the CRU3 gene.

Moreover, in order to confirm the presence or absence of targeting by genomic PCR, the following primers and the PCR reaction conditions shown in Table 4 were used. Forward primer: ctacgcgcatgaagatcaag (SEQ ID NO: 82) Reverse primer: tcctcgcccttgctcaccat (SEQ ID NO: 83)

[Table 4]

**Table 4:**

| Step | °C | Time | Number of cycles |
|---|---|---|---|
| 1 | 94 | 3 min | 1 |
| 2 | 94 | 30 sec | 35 |
| 3 | 55 | 30 sec | |
| 4 | 72 | 3 min | |
| 5 | 72 | 5 min | 1 |
| 6 | 4 | ∞ | |

### [Example 4] Confirmation of effect of improving gene targeting, using Arabidopsis thaliana (ATU6191 gene disrupted strain)

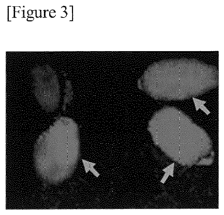

Arabidopsis thaliana was transformed using a mutant Agrobacterium strain (ATU6191 gene disrupted strain) into which the targeting vector produced in [Example 3] had been introduced, and the obtained T1 seeds were then recovered. Thereafter, T1 seeds, which emitted GFP fluorescence, were selected by microscopic observation (Figure 3). T1 seeds, which did not emit GFP fluorescence, were germinated on a hygromycin selection medium, and it was considered that, in hygromycin resistant plants, random insertion took place by non-homologous recombination.

Among 5198 T1 seeds obtained after the transformation treatment, GFP fluorescence was observed in 3 seeds (3/5198 = 0.06%). In addition, as a result of the genomic PCR, fragments obtained only when gene targeting took place were confirmed in these seeds.

Moreover, the percentage of the occurrence of random insertion in the obtained 5198 T1 seeds by non-homologous recombination was calculated, using hygromycin resistance as an indicator. As a result, the percentage was found to be 1.05% (45/4280). The gene targeting efficiency (homologous recombination efficiency/random insertion efficiency) of the present mutant Agrobacterium strain was calculated to be 5.5%, based on the above results.

### [Example 5] Confirmation of effect of improving gene targeting, using Arabidopsis thaliana (ATU6150 gene disrupted strain, ATU6188 gene disrupted strain, ATU6154 gene disrupted strain, ATU3081 gene disrupted strain, and ATU6156 gene disrupted strain)

The effect of improving gene targeting was confirmed by the same method as that in [Example 4], with the exception that the ATU6150 gene disrupted strain, the ATU6188 gene disrupted strain, the ATU6154 gene disrupted strain, the ATU3081 gene disrupted strain, or the ATU6156 gene disrupted strain was used as a mutant Agrobacterium strain. As a result, in all of the aforementioned five types of strains, the percentage of seeds, in which GFP fluorescence was observed, was 0.1 % or more.

### [Comparative Example 1]

The effect of improving gene targeting was confirmed by the same method as that in [Example 4], with the exception that a wild-type Agrobacterium strain was used. As a result, no GFP fluorescence seeds could be found in 5000 T1 seeds obtained after completion of the transformation.

## Claims

1. An Agrobacterium, which has a T-DNA nuclear translocation ability equivalent to that of a wild-type Agrobacterium, and has a T-DNA chromosomal insertion ability that is lost or reduced in comparison to a wild-type Agrobacterium.

2. The Agrobacterium according to claim 1, wherein homologous recombination efficiency upon its transformation in a plant is two or more times increased in comparison to a wild-type Agrobacterium, wherein the homologous recombination efficiency means the number of plants undergoing homologous recombination/the number of plants subjected to transformation.

3. The Agrobacterium according to claim 1 or 2, wherein gene targeting efficiency upon its transformation in a plant is two or more times increased in comparison to a wild-type Agrobacterium, wherein the gene targeting efficiency means homologous recombination efficiency/random recombination efficiency, the homologous recombination efficiency means the number of plants undergoing homologous recombination/the number of plants subjected to transformation, and the random recombination efficiency means the number of plants undergoing random recombination/the number of plants subjected to transformation.

4. The Agrobacterium according to any one of claims 1 to 3, wherein the gene targeting efficiency upon its transformation in a plant is 1% or more, wherein the gene targeting efficiency means homologous recombination efficiency/random recombination efficiency, the homologous recombination efficiency means the number of plants undergoing homologous recombination/the number of plants subjected to transformation, and the random recombination efficiency means the number of plants undergoing random recombination/the number of plants subjected to transformation.

5. The Agrobacterium according to any one of claims 1 to 4, wherein the function of a gene associated with T-DNA random insertion is lost or reduced.

6. The Agrobacterium according to claim 5, wherein the gene associated with T-DNA random insertion is one or more selected from among an ATU3081 gene, an ATU4309 gene, an ATU6150 (virH1) gene, an ATU6154 (virF) gene, an ATU6156 (virK) gene, an ATU6183 (virD3) gene, an ATU6184 (virD4) gene, an ATU6185 (virD5) gene, an ATU6188 (virE0) gene, an ATU6189 (virE1) gene, an ATU6191 (virE3) gene, an ATU6180 (virC1) gene, and a homologous gene thereof.

7. The Agrobacterium according to any one of claims 1 to 6, which is Agrobacterium rhizogenes or Agrobacterium tumefaciens.

8. The Agrobacterium according to any one of claims 1 to 7, which has a targeting vector comprising a foreign gene.

9. A method for producing the Agrobacterium according to any one of claims 1 to 8, which comprises disrupting a gene associated with T-DNA random insertion in the chromosome of an Agrobacterium by a homologous recombination method.

10. A plant cell which is obtained by infecting a plant cell with the Agrobacterium according to claim 8.

11. A method for producing a transformed plant, which comprises infecting a plant cell with the Agrobacterium according to claim 8.


SEQUENCE LISTING
<110>  Kaneka Corporation
<120>  Agrobacterium, and a method for production of transformed plant
<130>  141970A
160>  83
<170>  PatentIn version 3.5
<210>  1
<211>  1377
<212>  DNA
<213>  Agrobacterium A208
<400>  1
ttgcggtcag aagtaaccga acatgcggtg ccgattccgg ttggcccgct attgcactcc      60
agcgccgatc ttatggtctc gctcattgcc ttcaacaggc aggtgaaagc cgaacgtgaa     120
aaaccggcgg tgaccgccgt ctctgttgag aaggcgatga ctgagacgga caacaggatg     180
gataaggctg gaatggcggc cgcgccgacc atgccgccac ccatgtcaaa agacaaaccg     240
gccgacgtgg ccaataagag accctatctt ccccaacaga tattgctggc ggaggtcgat     300
gcgcggttta atggcaccgt ccgccagcct ctgatcgggt ttggcgaacg tcttgcgatg     360
ttctgggcaa accacttctc cgtcgccatc ggtaagggtg gcgatgtgca catcctcgcc     420
ggcgccttcg agcgcgaagc gatccgcccg catatattcg gcaatttcga ggacatgctg     480
cttgccgtgg aaacccatcc tgccatgttg ttctttctcg acaaccagca atcgatcggt     540
cccaactcac cggccaacaa aaacggcaag cgcggcctga acgaaaatct ggcccgcgag     600
atcatggaac ttcacacgct gggcgtggat ggcggttaca atcaggctga cgtgacggag     660
cttgcgcgca tcatcaccgg ctggaccgtt tatcgtgacg agaacaggcc ggggccggtc     720
gggcgcttca gcttcaatgc caacagccac gagccgggtg atcacgcggt gatgggcatc     780
acctatgccg atgccgggat ggagcagggg cggcgggcgc tgcgcgatct cgcccatcac     840
ccatccaccg cccgccacct tgccttcaaa ctggcaaggc acttcgtcag cgaccagccg     900
cctgtggcgc tggtgaccaa gatcgccgct gcctacacca aatcccacgg cgatctcgcc     960
gccacctatc ttgccatgct ggatgcggaa gaagcctggg atcccactct caagaagttg    1020
cggccaccgc tcgatttcgt caccgccgtg ctgcgcagcg gcgatgtcaa gccgaaacct    1080
gaacagatcg tttcggtgct gaaggcgctc gggcagcctt tctgggaccc ctccggaccg    1140
aacggttttt ccgatgtgag cgatagttgg ggttcctccg aggggctggc aacgcggatc    1200
gatgccgcga gcctttttgc ccatcaggtt gccggcggta tcgacccccg tgactttgtt    1260
gccgatcggt tcggcccgct tctcacgcgt gagaccgccc aggccgttca gcgcgcggaa    1320
accaagccgc aagggctgac catcgctttc ctttcccccg aatttcagag gcgctaa      1377
<210>  2
<211>  1032
<212>  DNA
<213>  Agrobacterium A208
<400>  2
atgacctctc ctcgcgccaa gaaacgcctc agcgtcgcct tcctgcttgc cgatcgcttc      60
acgctgtcgg ccttcgcgaa ttttgtcgat gtgctgcgcc ttgccgcaga cgaggccgac     120
cgctcccggc cgatcctgtg tgaatggtcg gtgctctcgg caacgctcgc caacgtgcag     180
tcgagctgcg gcgtgaaggt gcaaccggat acgcggctga tcgatgccgg acactacgat     240
tatatcgtcg ttgtcggcgg gctgatcagc gaccacagcg ccctgccacc ggaagcgcta     300
cgcttcctga aagatcgggc ggcggcgggc attccgattg tcggactctg caccggcgtc     360
ttcatcctgc acgaagcggg ccttctcgat ggctatcgct gctgcgtgag ctggtttcac     420
cgccaggatt ttctggatcg tttcgacacg gtgcaaatgg tctccgacca gatcttcgtc     480
atcgatcgtg accggctgac ctgttccggc ggccatggcg ccgcgcatct ggcggccttt     540
ctcgtcgagc gccatgtcgg ccagtccgcc gcgatcaaaa gcctcaacat catgatgatc     600
gacagcgcgc tgagcggcga aaaaccccaa cccggccagc agagcgcgcg caaggccaat     660
gaccccctgg tcaagaaggc gatcctgcgg atgcagcaga atatcgaggt tccgagaacg     720
gtgctggaac tggcccacga cctcggcctt ggccgccgta gcctcgaacg gcgtttcctc     780
aatgacctga aggcaacacc gtcaaaggtc tatctcgagc tccggctgga tcgcgccctg     840
tcacttctgc gcacgacgca tgacccgatc acccagatag cacttgccac cggcttctgc     900
gacgcgccgc atctttcccg cacgctcagg accgagcgcg gcttcacccc cagcgaatat     960
cgcaagaccc aggcgggcca cacggcaacc gacgaggaat tcgccgtcgg cgtgctgctt    1020
ccccaatcct ga                                                        1032
<210>  3

<211> 1260
<212> DNA
<213> Agrobacterium A208
<400> 3
atgatcacga gcagcatttc tgggaccgac cagcagtttc aaaacgccac gcagccaaaa 60
gagctcgatc ctgatgctgt tcctgtaagc aggcttgatt ctgaaggcca tgaaattttt 120
gctgaatggc gacctaagcg cccgttcctt cggagggagg acggcgtctt tttggttctc 180
cgcgcggatg atatctttct gctgggcacc gatcctcgta cgaggcaaat agaaacagaa 240
ctcatgttaa atcgaggcgt aacccgcggc gccgttttcg acttaatccg ctacagcatg 300
ctcttttcaa acggcgaggt ccatgtgaaa cggcgctcag cctttgcgaa gacctttgca 360
ttccggatga tcgacgcgct acgcccggag atcacgaagt taacggagca tttgtgggac 420
gatgtaccga gagttgacga ttttgacttt gctgaaatgt acgcgtcgaa attgcctgcg 480
ctaacgattg ccagtgtact tggtttgcca tttggggatg cacctttctt cacacgactt 540
gtttacaacg tttcgcgctg cttaagtcct tcgtggggag aagacgattt tccggagatt 600
gaggcttctg ccgtcgagct ccaggattat gtccgggcag tggtagcgga tcgcagccga 660
cgtataagcg atgacttcct ctcttgctac ttgaaagcgg tgcgggaaga aggaacgctt 720
tcacccatcg aggagatcat gcaattggtg tttctcatac tcgccggaag cgatgcaact 780
cgtaacgcga tggtgatgct gccgactctt ttgctgcaaa atcctgttgt ctggtcctct 840
ctatgccacg atcaatccgg cgtcgcggcc gcagtggagg aagggcttcg cttcgagcca 900
tcagttgggt ctttcccgcg gttggcgctc gaagatattg atctggatgg gtacgtattg 960
ccaaagggaa gtttcctagc cctaagcatc atgtccggcc tgcgggacga gagacactac 1020
gagcatcctc agctttttga cataaaacgc aaacaaatgc gcaggcacct cggatttggc 1080
gcgggtgtgc atcggtgcct cggcgaagct ctggcgcgga ttgaattgca agaaggactt 1140
agaacacttc tgcgccgcgc gccgagcctt agggtgacag gcgactggcc acggatgata 1200
ggtcacgggg gtgcacggcg cgccacgggg atgacagtca atctgggcgt cgatcggtga 1260
<210> 4
<211> 939
<212> DNA
<213> Agrobacterium A208
<400> 4
atggagccca gccaacgaag cttaagtgat gaatccgaag cgtcccgtac ggaagtccat 60
tctacatcga caaataggga tatgcctccc gagcttttgg ctaaggtggc aacttatatt 120
cctacccagg acccggtaga aacggcacga aatcttggaa gcctggaacg tacagggcgt 180
gcaggtcgtg aagccgttac aagtgatccc gtcggaaaat atcacgcgcg aatgaaacga 240
attggcgcct ccgccaaaac cgtgttcgat acggtcattc ccggaaatca attacctgag 300
tgggagacga accgaccctc tccgacggcc agaacgcgag ccgttggccc aattcttaag 360
ttccagtctg aagcgggaaa atcgagattt gtcaccaata tactcaattt gcctgaatct 420
gctcagtgcg acgcaattct tagtgtcata aaacacttga atgatcttgg tgaagcaaac 480
aagacgcgac ttatcgaacg ctctatcgaa attttgcggc tagaaccccc tttgacctgg 540
aatatgggcc agaaatgccc tgcggtcgac gtgctggtcc aaggcgaaaa gtacctaaat 600
gcggatcaac tggctcgcat acaacaagag aggagcagcc gtccgcggct gtctcccctg 660
tttggccgag ccatagccga tttcgaggtt caaaaaacca tggacgcaaa tcctagacga 720
tatagggacg cggcggggcc tgaggtcaga gctgtagaca cagtaattga gacaattgag 780
agatatagtg cgtcgttagc aagaggcggt ccaaatgcag tgggactcct aacgacgaac 840
gaaagctttg agaaaaatat caatgaggtc tacaaccgta cgcgagccga gctagacgct 900
tcttcacgtg acagaagtcg ctctggacta tcgcgataa 939
<210> 5
<211> 2502
<212> DNA
<213> Agrobacterium A208
<400> 5
atgaatggaa ggtattcacc gtctcggcaa gatttcaaga caggcgccaa gccttggtct 60
atcctggcct tggtagttgc tgcaatgatt tttgccttga tggcgattac gtcttggcag 120
gacaatgaaa ccaatcgggc gatcctgacc caattgcgag ctattaacat cgacagtgct 180
tcgctgcagc gggatgtact ccgcgcggaa gcgggtgtgg tggcgaacta ccggcccatt 240
atctccaggt tgggagcttt gcggaagaac ctggaaaatt tgaagcgact atttaaacaa 300
tctcatcttg tgatcggcaa tgatttctct caactgctcg acaagctaaa ggtgtctgtg 360
gatacgaccg acgcggccgt tgcagccttc ggagcgcaaa acgtgctcct gcaagattcg 420

cttgccagct tcactcgcgc gcttagtatt cttcccaaaa tgtcgtcgac ggatcagacg 480
gtcgaaaatt cgaacgaatt gggcagcctg atgctgcggt ttgtgcgtca gccaagccca 540
gcactctcgt tggagatcag tcacgaactc gacatgctcc aaaaagctag cggtggggct 600
gaagttccta tccgtatact tgcacgcgag ggtcgcgtca tcttgtcgat tttgccccgt 660
gtgaacgatg ccgtaaacat gattcagacc tccgacaccg ctgaaattgc cgaaagattg 720
gagcgcaagt gtttggaggc ctatagcttg caaagcgtga gggagcagcg ggcacggatc 780
ttcttgggtt ccgtttcggt gggcctttgc atctacatca tctcactggt ctataggctg 840
cgtcggaaaa cggcttggtt aacgcggcgt ttggattacg aagaggtaat caaagagatt 900
ggggtttgtt tcgagggggg aggggccaca gcgtcgtccc tcaattcgtc cgcgcaagct 960
gcgcttggaa ttattcaacg cttctttaat gcggaatcgt gtgcactagc attggtggac 1020
catggtgaca ggtgggctgt cgaaagtttc gctgcgaagc tgcctgagcc cgtctgggag 1080
gacctcgcgc tacgcgagat ggtttctctt gccagagcgg atgagcgtgc gtcagtattc 1140
cgcatcatgt cgacgcgaaa ggtcagctgc cttcctccgg agactccggg cgtttctatg 1200
ctgctggcac acaaatctac ggatcaactg atagcgattt gttccctcgg ttaccagggc 1260
tatcgtctga aatcttgtcc aggcgaagtt cagcttcttg aactcgccac cgcctgcctc 1320
tgccactata tcgatgtccg gcgtaagcag accgaatgcg atattctgga gaggcgatta 1380
gagcatgcgg aacgtcttca ggcagttggt acacttgctg gtggaatagc gcatgagttc 1440
aacaacattt tgggagcaat cctcgggtac gctgaaatgg cgcaaaacat gctgcgtcgg 1500
tcatctgtca cccgaagaca cattgaccaa attatttcgt cgggtgacag agccaggctc 1560
attatcgatc agatcttgac tctaagtcga aaactagaac gcgtgacaaa gccgttcagt 1620
gtctccgaac tcgtaatgga aattgctccc ttattgcgtg ttgcattgca gcgcaacatc 1680
gagctgaagt tcaagtttga cgacaagaag agcgtagtcg aaggaagccc gctagaggtt 1740
cagcagatgc tgatgaatct ttgcaagaac gcttcccagg cgtttaccgc cgatggtcaa 1800
atcgacatca tcgttagccg aatttttgta tctcgacaga aagtactggc gcatggtgtt 1860
atgccagctg gcgactatgt tcttctttct gtcagcgacg acggtgaagg cattgccgaa 1920
actgtgctcc cccacatttt tgagcctttc tttacaacac gctcttgcag cggcggcacg 1980
ggtctaggtc ttgctgccgt gcacggtcat gtcagcgcgc ttgcaggata tattgacgtt 2040
acttcagccg tagggcgagg gacgcgcttc gacatttatc tacctccttc ctcgaagaag 2100
cccgtcagcc cggacgcgtt ttttgggccc tgtaaaacac cgcgtggcaa cggagaaatt 2160
gtggcattga ttgagccaga tcctgtcttg cgggaggtgt acgaagacaa gatcgccgct 2220
ctgggctatg agccggtggg ctttaagaca tgtgcagacc tttgcaattg gatatcaaaa 2280
ggcaagcaag ccgatctggt tctagttgac caatcgtctc ttcccgagaa tcagagtgct 2340
actgctttgc acgcagcctt caagacggcg tccatcatca ttggaggaag tgatcttaaa 2400
atgtcacttt ccagcgatga catgacgtcc gcgctttttc tgcccaaacc tatatcctcc 2460
aggacgatgg cctacgcgat tcgtaccaag atcaaagcct ag 2502
<210> 6
<211> 2022
<212> DNA
<213> Agrobacterium A208
<400> 6
atggcaaatg aagagttcac cagacactat gcgtggcccg ttcctgtggc ttcgaatgat 60
gaagggcgtg ggaccgcgcg gatccccatc caggcacaat caatcgttgc tggagaagac 120
ggtcgggaca cttcggtccc aacggctttg tcgcgaccgc caattgaaga tatgccgcac 180
ggcgtccaag aaacatcggc gagtggcgga cgactgggag cggcccgtct gcgggattcc 240
gtaatcccgc caggaatatc cgaagcccgc acggacctat ccgcaatttt gcggaaaaaa 300
agcggttctt tccgcaccgg tatgcagtat ctgcgtgggc ttgaacggga aaattttgat 360
aaacaagaca gggaagccag tgcgttgcca gatttaagtg caaggggcat aaagcgaccg 420
cgcgaaattg agtatcccgg caatgcaagc ggattaacca taaagaggca agacggctta 480
ggcatagaga tcaatactat ctcggcatct tcgcccgtga accgggccgc gcattcgtcg 540
aactggcaag gcgcgccgga accgggcgtg tacaatgttc agccatcggc agatagagca 600
cagaactctg cgcaggaaag ttcgacgttt cccgatggca cttctgtttc cgctctctat 660
tcaggcccac tcgcagaatg gttcgagagg gatactggca gcgagacgac caggaattca 720
ggcaacacca tttcgtcacc gctccgaggg ctggaggagt tcggcgattc cgcggacagc 780
cggtatcttg ggcgcgaagc tcagagtctt tcagttaccg taacaacgcc taattcgaat 840
gctgaggcaa gttctcatag cgcacacact gaaactctcg acgatgtcag cagtgaccgc 900
tcgagcgaac aggggagggg tccccttggc gccgcaatcc ttggatccca tcatgacctt 960
tcaccgcgtg cgcagaagtt atcacaaaca aaccgcgatt cccctgaact gaccgacgcc 1020
gacctggcaa aagtcgatgc ggtgttcgaa tctctctcca aggggccccc ggcgggggag 1080

agcgccgcac cggactttcg tgagcgcgga ccaggcagcg ctttccagaa agaaggcgtg 1140
tccgacaggg cgaacggggt gcccactaac tgggaggtac cttttggtcg cggtggcggg 1200
cattcaccgc aagcgctgag gtcctcagga gtggagctcg atgacttccc tgatttcacc 1260
gaggccgaac tggcaaaaat cgacgcacta gtcgaatctc actccaacag atctttatca 1320
gtgcgaaata tagtaccaga tttgcgcgga gcgggagcag acaacgtctt ccggaaagaa 1380
ggcgttgtcg aacgcgcgga gaagatgccg atcgatagtg tgagtcttac cagactgaat 1440
ggcgagcgtt cacgttcacc gaagacatcg caagcaagcc ttgaggattt ccccgatcta 1500
accgatgccg atctggccca tatcgaagag tcggagcgga tcgcaagaac tgctgttgaa 1560
aaggggaagc aaaaaatctc gaccgaagcc gatacgcggt ttgatttggg caactcttct 1620
gcaccccggg ttagccctcg atcggttacc ccgttggttc ccaatgccaa tcagccgatt 1680
acatcgtggt tctacgaggc acaaaagact tgtgacaaat tggtagagaa cacttacgtg 1740
aagccggccg ttgacagttc tagggcgcga aatgacgtcg aaaacaccgc tgccaggctt 1800
ggtgaccctg ctcctgcttt ggggcatgac aatcttggta gaactcgcgc gttaacgccc 1860
gttcgcgacg tgatgtctag acccagcgcg gaccgacaac tggctagtca tgcggcagaa 1920
cattcggcaa tagacgacat ttggaaacgg gatgaccgtg atcgcaggac acacccttat 1980
agaggattgg attcgcgttc acgtgaaggt tgcgggcggt aa 2022
<210> 7
<211> 2007
<212> DNA
<213> Agrobacterium A208
<400> 7
atgaattcga gcaagactac gccccagcgt ttagctgtca gcatcgtatg ttcgctggca 60
gccggttttt gcgcggcaag tctctatgta acatttcgcc atggtttcaa tggcgaagcg 120
atgatgacgt ttagcgtctt cgccttttgg tacgagaccc cgctctatat gggtcatgcg 180
acccccgtct tctattgcgg tttagccatt gtcgtctcga cgtctattgt tgtgctgtta 240
agccaactta tcatatcgtt tcgcaatcac gagcatcatg gcacggctcg ttgggcggga 300
tttggcgaaa tgcggcacgc cggttacctg cagcgctata atcgtatcaa ggggccaatc 360
tttggcaaga cgtgtggccc tcgttggttc ggcagctatc tgaccaatgg cgaacagcct 420
cacagtcttg ttgtcgcgcc aacgcgcgcc ggcaaaggcg ttggcgtcgt tattccgacg 480
ctcctaacct tcaagggttc ggtgatagcc ctcgacgtca aaggcgagct ttttgagctg 540
acttccagag cacgcaaagc gggcggcgac gccgttttca agttctcgcc tctggatcca 600
gagcgacgga ctcattgtta caatcccgtc ctcgatattg cagctttacc gcccgagcgg 660
cagtttaccg agacgcgccg cctcgccgca aacctcatca cggccaaagg caagggagcg 720
gaaggcttta tcgacggcgc gcgggacctt ttcgttgcgg gcatccttac ctgtattgag 780
cgcggtacgc caacaattgg tgcggtctac gacttatttg ctcaacctgg agagaagtac 840
aaactttttg cgcacctcgc ggaagaaagc cgaaataaag aggctcagcg cattttcgac 900
aatatggcgg gtaacgacac gaaaattttg acttcttata catcagtgct cggcgacggc 960
ggacttaacc tgtgggctga tccattggtt aaagcagcga caagtcgatc agatttttcc 1020
gtctatgatc tgcgtcggaa gaggacctgc gtctatcttt gcgtcagtcc caacgacctc 1080
gaggtcgtgg cgccattgat gcgcctcctt tttcagcagg tcgtgtcaat tctgcagcga 1140
tcactgccag gtaaagatga gcggcatgaa gttttgtttc tccttgacga atttaaacac 1200
ctggggaagc ttgaggcaat cgagaccgcg attacaacca tcgccggtta caaaggccgc 1260
ttcatgttta ttattcagag tctctcggcc ttgacgggca tctacgatga cgcgggcaaa 1320
caaaactttc tcagtaacac tggcgtgcaa gtatttatgg ccacggccga cgacgaaaca 1380
ccgacctata tctcaaaagc tatcggcgac tatacgttca aggcgcgttc gacctcatac 1440
agtcaagccc gaatgttcga tcataacatc cagatttccg atcagggggc accgcttttg 1500
cgccccgaac aagtgcgctt gcttgacgat aacaatgaaa tcgtccttat taaagggcat 1560
ccgcctctca aactacgaaa ggtgcgatat tattccgatc gtatgctgag gcgccttttc 1620
gaatgccaaa ttggcgccct ccctgagccc gcatctttga tgctttcgga aggtgtccat 1680
cgggatgggc aagacctcag tcaacaagcc gccgtcacgg aggcgcaggg cttaggtgac 1740
atcgattcga tacctaataa tatggaagcc gctacaccgc aaaacagtga aatggatgac 1800
gagcaggaca gcctcccaac tggaattgac gtcccccagg gccttattga aagcgatgag 1860
gtgaaagaag acgccggtgg cgtggtgcca gatttcggtg tttcagctga aatggctcca 1920
gctatgattg cacaacagca gctgctggag cagatcattg cgcttcagca acgatatgga 1980
cccgcgtcct cgcactcggt gaaatga 2007
<210> 8
<211> 2523
<212> DNA

<213>  Agrobacterium A208
<400>  8
atgagacctt caggaaaccc gaacgtcgat cttagcggtt cgactgcatc gcttgccgaa      60
gttcccgccg gagctacccc tgtccttaat ctaatcgagc ccaggaaccg tccggctgac     120
gactcgcttg agggccaaac cgatcgcggc gagcatccat ctgcatcatt tgactatgat     180
ggcatgaagc ttggcgccgc ggagcgtgaa gcatacgaga actggtgtcc atcgaaccgg     240
cctacatgga aagatctggt actcagggcg cgccttgatg caatcgacag ttccgcttgg     300
ctccccgatt tgggcgagga gtcgcctttg atcttcagat atgaagggat tccgctgggt     360
gagggggaac ggcaagccta caaagaatgg caagaggagg ctcagcccac atgggaagac     420
ctcgttgtca acgcacgaat ggcggtacct gatccttgtg ctgacgttgc agacgagcac     480
aatcccctca aagaaggcga ggagtttcgg tctgaagcgt cgaaacgcaa gcggaaaaaa     540
ccgatcgacc aggacgagaa ttctcctaca tcgttttact atgacgggat gaggctcgga     600
gaacccgagc gcgaggcata tgataactgg ggcaacgcgg agccgcccac gtggaaagac     660
ctggtactta aggcgcgcct tgatgcaatt gacagctccg cctggctctt tgcttcagaa     720
gggtcttcct cgacttttga gtatgaggga attccactgg gtgaggggga acggcaagcc     780
tacaaagaat ggcaagagga cgctcagccc acgtgggagg acctcgtcat taatgcacgc     840
atggcagaac tcgaccatcc ttcttggatt acagacgagc acaattccct tgaagaaaac     900
ttagagtttc ggcccgatgc aagacaggcc agcctgaagg actcgaccga ccagcggaag     960
agttcttccg cgtcatttat ctatgatgga atgaagctcg gggaacccga gcatgctgca    1020
tacgagaact ggagcaaacc ggaacgaccg tcatgggaag ccctcatcct agatgcgcgc    1080
caggcttcca tagcaagctc ttcggtttcg aattcgttac ttgcaaagac atcctcgcca    1140
gtctttctat acgagggaat gtcgctaggg gatgcggaac gtcaatcgta tggaaggtgg    1200
aggcagcctg cccaaccgcg atggcaaaat cttgtggtga acgcgcgcct tgcggatctt    1260
gatccgttgg cctggattcc cgatgaacat gatccgtttg cggaagccga ggcgcttagc    1320
cccacttcgc aatcgagtgg cgccaacaag tccaaccgcg ctttgggtca atcagattca    1380
ggccggcccg ccttcgcata tctggcagca caagaggcga gtcacctgca atcaccggca    1440
tgctcacaat tggaaacgag gcgtgcatta aatttcgggt cgcctggacc ggatgcaaat    1500
ccaacggaaa gcatcgccaa atgtaatcgt ttggatggcg tcagtaaaat taagcgatta    1560
ggcaccaaag gccgccgggc agtaaacgcg actattcacg gcggcaagtt cggtgcgcaa    1620
ggactgttgt ccgaagattg cggacaagcg gctgagccct ctccatcgga acaaactact    1680
cggccgcgaa ccgataatat tggtacctat gcaagccgga aaaacgaacg agctcggctg    1740
gctacagaga ccgggaaata cgaatcggag cacattttcg gatttaaggt cgtccacgat    1800
actttgcggg cgaccaaaga aggccgtcgt ctcgaaaggc caatgccggc atacctcgaa    1860
tacaaggagc ttcatcggca acacgtaggg acaggaagag gacggactgg actggtcggg    1920
cgcggatggc cagatgatgc gagctatcgc gcagatcaaa gggcaaccct gtcggaccct    1980
gttgcgtccg cggaaggtgc aacagcttca aatgggtacc agttgaacca gctgggctac    2040
gcgcaccagc tcgccaccca tggtctccaa agtgaaacgc ccgatggggt taccatgcca    2100
attcaagttg cgacaattag ctacaactat acagtgagtc gtgatccggt cctttcacca    2160
cccagcaaag aacaagcgcc cccattgcta caccttgggc ctcgtgggca aacggaagct    2220
gtgcttgccc gcgagaccgc attgaccgga aaatggccaa ctctcgagcg tgagtgtcaa    2280
gtgtatcagc ggtttttggc cctgtacgac gtaaagaagg atctggacgc caaacaactt    2340
ggtgtacggc agaaaaagca ggcactcgtt gcggcgttga accggaccgc cggcttaatt    2400
ggcgcgtcac ctttggaagc ccaatcgtcg accgctgagg ttgaatatac taccgacgag    2460
cccgatgaac gacgggttta tgatccgcgc gatcgaggca gagacaaagc gttcaatcgc    2520
tga                                                                  2523
<210>  9
<211>  252
<212>  DNA
<213>  Agrobacterium A208
<400>  9
atgcacggtg atgatgttga tcggacggct ttcgtcccgc gcaggctgcc ggctgaagcc      60
gcttacgtcg atagtcaagt tgacctggcg gcaaaccgac aacctcactg tgtgacttgt     120
tttgagcgac ttttggttca agagaaagat gaaaaccgtc gacgattgtg ggcttccggc     180
aaagcatgcg ccacgtctga agagagttgt gcaggaggcc ctacactcgc accgggccat     240
tcggggccat ga                                                        252
<210>  10
<211>  192
<212>  DNA

<213>  Agrobacterium A208
<400>  10
atggtgatca tcaagctaaa tgcgaataaa aacatgcctg tcttggcggt tgagaagccg      60
caagaaattc acaaagagga gttgagcgac catcaccagt caaatggctt tacgagtttg     120
gatctcgaaa tgattgaact ggagaatttt gtccttcact gcccgctccc cgaagaaaac     180
ctagccggct aa                                                         192
<210>  11
<211>  1671
<212>  DNA
<213>  Agrobacterium A208
<400>  11
atggatccga aggccgaagg caatggtgaa aatatcactg agaccgcagc aggcaatgtc      60
gaaacttctg atttcgtgaa tttgaagcgc cagaagaggg agggcgtaaa ttccaccggg     120
atgtccgaaa ttgatatgac gggtagccaa gaaactcccg aacacaacat gcacggaagc     180
ccgactcaca cggatgatct cggcccgcgg ttggatgcgg acatgctcga ttctcagtca     240
agtcatgttt ctagcagcgc tcaaggcaat cggtctgagg ttgaaaatga gctatccaac     300
ttattcgcga agatggcttt accaggccat gatcggcgta ccgacgagta tattcttgtg     360
cggcaaaccg gacaagacaa gttcgcaggt actactaaat gtaacctcga tcatctgccc     420
accaaggcgg aattcaatgc gagctgccgg ctctataggg atggagtcgg caactactat     480
cccccgcccc tcgcattcga gaggatcgat atcccggagc aattggctgc acaattgcat     540
aacctggagc caagagaaca gagtaaacag tgttttcagt acaagttgga agtctggaat     600
cgcgctcacg cagagatggg catcactggc accgacatct tctatcaaac agacaagaat     660
attaagctcg accgaaatta taaattgagg cctgaggata gatatataca aacagagaaa     720
tacgggcgca gagaaattca aaaacgctat gagcaccagt ttcaagctgg ttcactgctg     780
ccggatatct taatcaagac cccgcaaaat gatatacatt tctcgtacag gtttgcgggc     840
gacgcttacg ctaacaagcg atttgaggaa ttcgaacgcg caatcaaaac taaatacggt     900
agcgataccg agatcaagct caaatccaaa tctgggatta tgcatgactc caaatatttg     960
gaatcatggg agcggggcag tgcggatatc cgtttcgcag agttcgccgg cgagaatcga    1020
gctcacaaca agcagtttcc ggctgcgact gtgaatatgg gaaggcagcc agatggccag    1080
ggagggatga ctcgcgatcg ccatgtaagc gttgactacc tattgcaaaa cctacccaac    1140
tccccttgga cgcaagcctt gaaagaggga aagttgtggg atcgagttca ggtccttgct    1200
cgcgacggaa accgttacat gtcaccttca agactggaat attccgaccc cgaacacttt    1260
acccaactga tggatcaagt tggtctgccc gtgtcgatgg gtcggcaaag tcatgcgaat    1320
agtgtcaagt ttgagcagtt tgacagacag gcagcggtta ttgttgcgga tggcccgaac    1380
ttacgtgagg ttccagattt gtccccggaa aagttgcaac aactgtctca aaaagatgtc    1440
ctgatagcgg atcgcaatga aaaggggcaa agaaccggca cttacactaa tgttgtggaa    1500
tatgagcgcc tgatgatgaa attaccgagc gacgcagcgc agcttctcgc tgaaccgtcc    1560
gatagatatt cacgtgcttt tgtccggccg gagccagcat tgccccccat cagtgacagc    1620
cggcggactt atgaaagccg accgcgcggc ccaaccgtaa acagtctgta g              1671
<210>  12
<211>  2055
<212>  DNA
<213>  Agrobacterium A208
<400>  12
atggtcgaca cgacgaagaa gagtgtcgcg aagtcgctta cggctgacat gcgccgttct      60
gctaagcggc tttctaagca aatgcgtaaa gcctcgctta ctgaagagga ggcaacaagg     120
aatctagccc ggctcgaaac gccggaccag aagcgaaaat atgtcgccga tatgcagata     180
atcgacaagc tggaagacgg ctttcgaggc gaaataagct ataaaatgct gggaaataaa     240
cagcttcggg tcgacagccc aaaagaatta acgcgcgagc atggtataat aagaaaaaca     300
agaaaggttc tgaagcgtaa cgcagagact ggcaatgttt acttgggtct ccacgaaaag     360
aagacctgga ggagcgttag cagccatctt tatgccgagg acggtacact tcgcgcgaag     420
catgtgaaat acaaagacgg acgctttgaa gaaaaatggg aacgagacga aaatggcctg     480
ctgttccgca cgcagtttgt caaccgaaat cggctatttc aacctatttc cgagaaggtc     540
agcacaccct accggagcgg accggaaaac cggctctttc gtgaactaac ccgtcgaaaa     600
ggttccaagc aggaaacttt tgagcgggac gaaaaaggca acctcgagct catcggcagc     660
aaacgtctcg gcttttccaa gaattcgacg aaagccgtgg atcgtcagac ctctcagacg     720
acgattcgaa aacttggtgg cgcattcagc aaatcttata gatccctgct agacacagag     780
ggcaacgaac ttggccgaga tatatcgagt catcgacggc tcttcaacaa gcgatcggct     840

gtctacgatg aggctagcgg gcaattgacg agcaccaagc atacgttcgg taagatctac 900
aagagtgaaa cagcgtattt gaacgctgat atcaaaaaag tctcaaaaaa gatactcggc 960
gtgacggttc gtcggaaact gacgacgcta agtgaacaag aacacgacgc tcagaagctg 1020
cgaaattcgg aatccattga gcataggaag gcttggcaag agcgcgcagt tatccctaga 1080
tcgcctccac gggagacggc aaatgtggat tcggcgccgc agtcgcatct ggtcaactct 1140
cttagagacg gccgtcgtga tgaggctgag cctagggttg tgcctgcaaa agaccgacga 1200
ttagaaggcg ttatacaaaa ttcacccttg tttcggcaac cgaactccga gcgacgggtc 1260
ggttcccaaa cactacctcc atcgtcaaat gtgcgtgcct cagatccagt ttttccaagg 1320
gaaggcgcta agtcggaaaa agggccagtg gcggtcaatc tccagcgtga taatgaaagg 1380
gaaaagcaag aggaaaaaaa cctcagtgag aggcggggaa atctcttccg gtcaagccgg 1440
tcaataacag agacgtcgtt tcccggatcg ccggaaagaa taacgatgtt aggttcgcgg 1500
cgggcctcga taaacccatc cgccgatccg caatcgccgg tcggcagaac ggattcccaa 1560
gcgccgccga tgtccttatt tccgatcaat aatcatcagt cggatccaaa cgagcaagag 1620
ctcttgagtt tcctgcatag cgtgccagtc ccgccgcctt tggaagtaca tggcgcacca 1680
ggcgggcgtg ttgaggacag cctccgtgga gcttccggag acagcctggt gcgaacgagt 1740
tcagtgtcgg agagtgtgtt cgatgaacat tcgcaagggc ctttggaacg cgatcactcg 1800
accaatgcga caagtgagcg ctttgatccg caggctttgt ttggcgaacc gggcttgtcg 1860
cgcgtctcag aaacacgacc agaactctcg atgcaaggcg accatttgac aaattcggaa 1920
cagcaagcgt tactgaatga actgcttagt gtgccattac cgggtccttt gccgaaggcg 1980
gatcatgagc gaccgagggt tttggaaagc tcacggagcc gagagcgctc catgtcagga 2040
gggctttcac tttag 2055
<210> 13
<211> 435
<212> DNA
<213> Agrobacterium A208
<400> 13
atgaaactta tgctacccac cctgtttggg atacttttgg ctacggctgc aaacgctgcc 60
aatgatagtc atgtactagc aacctttccc gagattgcgc ttgcactgac taccggcaag 120
ccagtggacg ttatggtcga tttaagtttg tgtactccgg ggaccgacga tacaccgccg 180
acgaagacgc gcggaggcat tcgcatagat gggtatagaa tcacttctga cggcaccctc 240
gcttttgcgg atcagcactt tacgattgat cgtgacggta agcctattct tcagtttctt 300
cgctaccaaa tccggttgga cggtgacgcc gaactgacta tggtcgtatt caacatgcca 360
agctacgaga gaaaaggtac cagcttggcc tacaagtgcg cgatcagtca cgggttgagt 420
ttcttcgctc catag 435
<210> 14
<211> 696
<212> DNA
<213> Agrobacterium A208
<400> 14
atgaaacttc tgacattttg ctccttcaag ggaggagccg gcaaaaccac ggcactcatg 60
ggtctttgcg ccgcctttgc aagtgacggc aaacgattgg ctctcttcga cgctgatgaa 120
aaccgaccac taacgcgatg gaaagaaaac gcccttcgca gcaatacctg gggctccttc 180
tgcgaagtct acgccgccga ggaaatggca ctccttgagg cggcctatga ggacgccgaa 240
ctccagggat ttgattatgc gctggccgat acgcatggtg gttcgagcga actcaacaac 300
acgatcattg ccagctcaaa cctgcttctg atcccgacca tgttaactcc gctcgatatc 360
gatgaagcat tgtcgaccta ccgctatgtc attgaactgc tgctgagcga gaacttggca 420
attccgacag ccgtattgcg ccaacgcgtg ccggttggtc gattgaccac atcgcagcgc 480
gcgatgtcgg acatgctcgc aagccttcca gttgtacagt ctcccatgca cgagagagac 540
gcatttgccg cgatgaagga acgtggcatg ttgcatctca cattgctgaa tatgagaacc 600
gatccgacaa tgcgcctcct cgaacggaat ctcagaatcg ccatggagga actcgtcact 660
atctccaaat tggttagcga agccttggag gggtga 696
<210> 15
<211> 1269
<212> DNA
<213> Agrobacterium EHA101
<400> 15
atgattgcga acagctcttc cgatgtctct atggccgacc agaagtttct aaatgtcgcg 60
aagtcaaatg aaatcgatcc cgacgccgtt cctataagca gacttgattc tgaaggtcac 120

agtatttttg cagaatggcg accgaagcgc ccgtttcttc ggagagaaga tggcgtcttt 180
ctcgttctcc gcgcagacca tatctttctg ctgggcaccg atccacgcac ccggcaaata 240
gaaactgagc tcatgctgaa tcgaggcgtc aaagctggtg ccgtttttga ctttataggt 300
cacagcatgt tgttttcgaa cggtgaaacg catgggaagc ggcgctcagg tctttcaaag 360
gcgttctcat tccgcatggt tgaagcgtta cgcccggaga ttgcgaagat aacggagcgt 420
ttgtgggacg aactacaaaa agttgacgat ttcaatttta ctgaaatgta cgcgtcgcaa 480
ttgcctgcgc tgacgatcgc aagtgtcctt ggcttgccgt ctgaggacac gccgtttttc 540
acacgacttg tttataaggt ttcccgctgc ttgagcccgt cgtggcgaga tgaggaattc 600
gaggagattg aagcttccgc tatcgagctt caggattacg ttcggggcgt gatcgcggat 660
agtggccgtc ggatgaggga tgattttctc tcgcgctact tgaaggcggt acgggaagcc 720
ggaacgcttt cgcccattga ggagatcatg caactcatgc ttatcatact cgctgggagc 780
gatacaacgc gcactgcaat ggttatggtg acggctcttg tgctccaaaa tcccgcgctc 840
tggtcttctt taactggcaa tcaatcctat gtcgcagccg ccgtggagga agggctccga 900
ttcgagccgc cagttggctc ttttccgcgg ttggccctcg aggatatcga tctggatgga 960
tacgtgttgc caaagggaag cctcctcgcg ctcagtgtca tgtctggcct gcgagacgaa 1020
aaacactacg agaaccctca tctttttgat gttgggcgtc aacaaatgcg ttggcacctc 1080
gggtttggcg cgggagttca tcgttgcctc ggcgagacgt tggctcggat tgaattgcaa 1140
gaaggacttg gaacactttt gcgccgcgcg ccgaatctta cggtggtagg tgactggcca 1200
cggatgatgg gtcacggagg catccggcgc gccaccggca tgacggtcaa attgagcgtc 1260
gaccggtga 1269
<210> 16
<211> 942
<212> DNA
<213> Agrobacterium EHA101
<400> 16
atgcagaata ttgtatccaa ttcacagaca cagcggcaac ccatagaaaa agaggtgtca 60
cccttccatc gcgtgatgag cgacgatctg atctcccgga tcgtggatgg catggtcact 120
aacgatcctg ttgagactgc aaggaatatc tcgaacctta agctctctaa taaatctgta 180
aaggaaagtg ttgagcgtag tgctggagga agatttcacg gacaaatcaa tagacttggc 240
gcagcaagca aggccttata tgaccacgcg gttcccacag aaggattctc tgagcatcct 300
gagtttcgtc cagccttgcc aagggactac gccctggccg gggagagaat tgatgcaatc 360
gggggaactt taaagctgca aacaccgcaa cgaaaatcag cgctcgtaga tcatatattg 420
aatatcgcag aaccgtacga tcaaggtcac gcgcttgagt atgttgcgcc ccatgtcggt 480
gaatttagct cggaagatcg tcgacgtctt gtcgagaaaa tgcttgagca cttttccacc 540
ccctttaccg atgagttgcc aagcgatgaa aacttcagtg gaacttacgc cttgttcaag 600
gcattgcccc acatggaaga tagcctgaag gcgaggatgc ttgataccgt ggtcaataat 660
cctgatatgg cagcactcat tcaatcgcag aaggaacgct atgattggtt gagggcgcaa 720
gatgagtacg agaaggccac ttggcgctcc acgcaaaagc caactgtcga tgagcggctg 780
gcagacatag aaagttcagt tagaagcaat ctcagcctcg gtcaaggcag tgaccttgag 840
aagatgcgct ctgcaaggcc cctgcaacag actataagtg acaccctcaa tctcgcgcgt 900
gaagaactca aaagccgcgc ccccgacgtc cgagggaggt aa 942
<210> 17
<211> 2498
<212> DNA
<213> Agrobacterium EHA101
<400> 17
   taagtgcgat gaacggaagg tattcaccga cgcggcagga ttttaagaca ggcgcgaagc 60
   cttggtctat attagccctt atcgttgccg caatgatttt cgcgttcatg gcggttgcgt 120
   cctggcagga caatgggact acccaggcaa tccttagcca aatacgatcg attaacgctg 180
   acagcgcctc actgcagcgc gatgtactcc gcgctcacac gggcaccgtg gcgaactacc 240
   gccccattat ctccaggctg ggagctctgc ggaagaatct ggaagatttg aagcaattat 300
   ttaggcaatc tcatattgta agtgagagca atgctgctca actgctacgc cggctagaag 360
   tgtctctaaa ttcggctgac gcggcggtcg ccgcctttgg tgcgcaaaat gtccgcctgc 420
   aagattcgct ggccagtttc actcgtgctt taagcaatct tccaggaaag gcctcaagcg 480
   atcagacttt agaaaaacca acagaattgg ctagcatgat gctccaattt cttcggcaac 540
   caagcccggc tatttcattc gagatcagcc ttgaactaga gaggctccaa aaacaacgcg 600
   gtcttgatga agctcccgtg cgcatacttt cgcgtgaagg tcccattatc ttatcgcttt 660
   tgccacaggt gaacgatctg gtgaacatga ttcagacgtc tgacaccgca gaaattgcgg 720
   aaatgttgca gcgtgagtgt ttggaggtct atagcttgaa aaatgtggag gagcggagcg 780
   cacgtatctt tcttgggtcc gcttcagtgg gtctttgcct gtacatcatc accttagtct 840
   ataggctacg caaaaaaacc gattggttag cgcggcgttt agattacgaa gagctaatca 900
   aagagatcgg agtatgtttt gaaggtgagg cggccactac gtcgtccgcg caagctgcac 960
   ttggtattat tcagcgcttc tttgatgccg atacgtgcgc gttagctcta gtggaccatg 1020
   accgtaggtg ggctgtcgaa acattcggtg cgaagcaccc aaaacccgtg tgggacgaca 1080
   gggtgctacg cgaaatagtc tctcgtacca aagcgaacga acgggcgacg gtattccgca 1140
   tcgtatcgac gcaaaaaatc gtacatttgc ctcccgaaat tccaggtctt tcgatactac 1200
   tggctcacaa atccacagat aaactaattg cggtttgttc actgggttac caaagctatc 1260
   gccctcgacc ttgccaaggc gaaattcagc ttcttgaact cgccaccgcc tgcctctgtc 1320
   actatatcga tgttcggcgt aagcagaccg aatgcgacgt tttggccaga cgattggagc 1380
   atgcgcaacg ccttgaggca gttggtacac ttgccggcgg aatagcacat gaatttaata 1440
   acattttggg gtcaatcctc gggcacgcag aattggccca aaactcggtg tctcgaacat 1500
   ctgtcacccg aagatacatt gactatatca tttcgtcagg cgacagagcc atgctcatta 1560
   tcgatcagat cttgacgctg agccgaaaac aagagcgcgt gatcaagcca tttagtgtct 1620
   cagagcttgt gaccgaaatc gctcccttgc tacgtatggc tcttccccca accatcgagc 1680
   ttagtttcag atttgatcaa atgcagagcg tgatcgaagg aagcccgctt gaacttcaac 1740
   aggtactaat taacctctgc aagaatgctt cccaagccat gactgcaaat ggtcaaatcg 1800
   acatcttcgt cggccaagct tatttaccag ctaagaaaat tctggcgcat ggtgttatgc 1860
   cacctggcga ctatgttcta ctatctgtca gcgacaatgg tggaggcatt tcagaggctg 1920
   tgctacccta catttttgaa cccttcttta cgacacgtgc tcgcaacggt ggaacgggtc 1980
   tcggcctcgc ttctgtgcat ggtcatatca gcgcgtttgc aggttacatc gacgttagtt 2040
   caactgttgg gcatgggacg cgctttgaca tttatctccc tccgtcttct aaggagcccg 2100
   tcaatcccga cagctttttc ggccgcaata aggcaccgcg tggaaacggg gagattgtgg 2160
   cacttgttga gcccgatgac ctcctgcggg aggcgtatga agacaagatc gccgctctgg 2220
   gatatgagcc ggtcggtttt cgtaccttta gtgaaattcg cgattggatt tcaaaaggca 2280
   atgaagccga tctggtcatg gtcgaccaag cgtctcttcc tgaagatcaa agtcctaatt 2340
   ccgtggattt agtgctcaag accgcctcca tcatcattgg cggaaatgat ctcaaaatgc 2400
   ccctttcaag ggagaatgcg accaggaccc tttatctgcc gaagccgata tcgtccagaa 2460
   ctatggcgca tgcaatccta accaaaatca agacgtag 2498
<210> 18
<211> 639
<212> DNA
<213> Agrobacterium EHA101
<400> 18
atggcaaatg gtcagttcac gatacgctct gctcgcccgg cctccgtcgg actgacaggc 60
gaacggcgtg gagccgcatc cgcctctagc tctgcactgt ccaatgttca aagagatgtt 120
agggataggc tgattccaac tagctcacca agattaccaa atgcagccat attgcgtgat 180
tcctcgggaa gagcgtcgac tggtctgcgg tacatggcgg ctactcttca ttggtctgcg 240
atcgcgccat tatcgctaat aaacagcaac gacctggctc cggccgctta tgactttgag 300
acgcgaaata acgcaagaaa tgtgactgcc aaagtcggca gggcagtccc tgttcccaag 360
caaggcgggc tcggcaaaac gctcgcaccc gtacccctta gtacacgtat atcaagggtc 420
aattccgacc gaagactgcc cgctgacgca gaagaccgcc ctgaaacgcg cgacccccag 480
aaaggacgtg gcagtcatgg tgcgacgcca accttacatg aaaagattgg aaccgcgttt 540
gctcgaagat tgcgaaagca tacgtactat attgtttgca gttgctgcca gacccggagc 600
gcgttgacga tgggtgcaaa gatttcggtg aagtcatga 639
<210> 19
<211> 1959
<212> DNA
<213> Agrobacterium EHA101
<400> 19
atgaactcca gcaagacttc gccccagcgt atgaccctga gcatcgtatg ttcgctggca 60
gccggttttt gtgcggccag ctgctatgta acgttccgcc ggggcttcaa cggcgaagcg 120
atgatgacgt tcgacgtttt cgctttttgg tatgagaccc cgctttactt gggttatgcc 180
agcaccgtct tctggcgtgg tttatctgtt gtcatcttta cctcgctgat cgttctttca 240
agtcagctca tcatatcgct gcgcaatcag aagcatcatg ggacagctcg ttgggcagaa 300
attggcgaaa tgcggcatgc tggttatctg cagcgttaca gtcgcatcaa ggggccgatc 360
tttggaaaga catgtggtcc cctttggttc ggcagttatt tgaccaatgg cgaacagcca 420

cacagtcttg tcgtcgcgcc aacgcgtgct ggcaaaggcg tcggcatcgt cattccaacg 480
ctgttgacct tcaagggctc ggtaatcgcc cttgacgtca agggagaatt gtttgaactg 540
acgtccagag cacgcaaagc gagcggcgac gcagttttca agttctcccc cctagatcct 600
gagcggaaga ctcattgtta caatccggtc ctggatattg ccgcacttcc gcccgaacgc 660
cagttcactg aaacacgccg tctagctgcg aaccttatta cggctaaggg aaagggagca 720
gaaggcttta ttgacggcgc acgtgacctg ttcgtcgcgg gaatccttac ctgcattgag 780
cgtggcacac caacgattgg cgcggtatat gacctatttg cgcagcctgg cgaaaagtat 840
aagctttttg cgcaactcgc ggaggaaagc ctaaacaaag aggctcagcg tatcttcgat 900
aatatggcgg gcaacgacac gaaaattctg acatcgtaca cctctgtgct gggcgacggt 960
ggactgaacc tgtgggttga tccgcttatc aaagcagcga caagccggtc agacttttcc 1020
gtttacgatc tccggaggaa gaagacctgc atttatcttt gtgtcagtcc caacgatctg 1080
gaggtcttgg caccacttat gcgcctgatg tttcagcagc tcgtgtcaat cttgcagaga 1140
tcgctgccag gtgaagacga gtgccatgaa gttttatttc tcctcgacga attcaaacac 1200
ctgggcaagc ttgaggccat agagaccgcg atcacaacca tcgccggtta cagaggccgc 1260
tttatgttta ttattcaaag tctttcggcc ttgtcgggca catacgatga cgcaggaaaa 1320
caaaactttc tgagcaatac tggcgtacaa gtatttatgg ccacggctga tgacgaaact 1380
ccaacctaca tctcaaaagc tatcggcgaa tatacgttta aagcgcgttc gacctcttac 1440
agtcaagcca gaatgttcga ccacaacatc cagatttctg atcaaggtgc agcccttttg 1500
cgccccgaac aagtgcgcct gctagacgat cagagtgaaa tcgttctcat caaaggcgca 1560
cctccactca aattacgaaa ggtgcagtat tattccgatc gtacgctgaa aggccttttc 1620
gaacgccaga tgggctctct gcctgagccc gcacccttga tgctttccga ctatagcaac 1680
gatcaagttc aataccactt ggctccgata gcaaatttta atgaggatgc tgcaccgcaa 1740
aacagaactg tggccgagga ccatggaagt gttaaagtcg gtgctgatat ccctgaacgc 1800
gtgatgggaa taaatggtga cgaggaacaa gccgatgcgg gcgagatacc gccggaatcg 1860
gttgtgcctc cagaattgac gctcgctctg accgctcaac agcaattgtt ggaccagatt 1920
attgcacttc agcaaagatc gaggtccgca ccggcatag 1959
<210> 20
<211> 2511
<212> DNA
<213> Agrobacterium EHA101
<400> 20
atgacaggaa agtcgaaagt tcacataaga ggttcggctg acgcgcttcc tgacgttcct 60
ggcggaagta ctaccgcccc ttttttaacc gaaccttctc gggatcaggt tgatgcctcg 120
tttgaggtcc aaaccgacta cagccagtct acttccgtgt cgtttaccta tgatggtgtt 180
ggacttggtc ctgccgagcg tgcggcttac gagaactggt gcgaaccggg ccggcccact 240
tggaaagatc ttataatcaa ggcacgtgtc gatccgattg acgatgtgac ctggctccga 300
gatttagaag aggacacccc ctcaaccttc agatacgaag ggatgcctct gggcatcggg 360
gaacgacagg cctacgaaaa ttggcaagag gacgctcagc cgacatggga agaccttgtt 420
gtcagcgcac gcttgacgga acttggccgt ccacacggga ttaccggcga gtatacatcc 480
ctcgcaggat cgaagaatac aagttcaatt tcattgaagc gaaagcggag caacttaatt 540
gatgatgaga attcatccgg atcgttttca tatgacggga tgaagctcgg ggaagccgag 600
cgttctgcat atggtgactg ggccgaggcg gagccaccca cgtggaaaga tttggtattg 660
agggcacgcg tttcctcgat caatgactct gcttggcttt ttgattcaca aacatcttca 720
tcatcatttg aatacaacgg tgttcccttg ggcgagccgg aacggcaggc tctcagacaa 780
tggcaaggag acgctcagcc tacctgggaa gatctcgttg ttaacgcgcg tatggcagaa 840
ctttgccatg ctggttggat tgaaggtcaa aaaggttgct ttgaagagcg cggggaggct 900
ctgcccgcgt cggaacgcgg ttcgcaacgc cccattggtc aacggacaga ttcctccgat 960
tcttttgtgt atgatggcac aaggctcgga gcacctgagc gaactgctta tgaacgctgg 1020
agtaagaggg aacgcccgac ttgggaagat ctcatcttag atgcacacca ggccaggact 1080
gaaagtgacg ctgttacgac ccaagcgatt ggtcagtcgt cctcaccggt tttcttatat 1140
gaaggaaagt cgctcggaga cagggaacga aaggcttacg aaaaatggcg gcagccagcc 1200
caaccgcgat ggcaaaatct tgtagtgaac gctcgtctgg cagaaatcga tccctcagcc 1260
tggattgccg atgagcgcga tccgcttgat gatagcgacg cgcttggtcg cccgtcgtac 1320
acaagcttga cggatagatc agacgtccct ttagacgatc aatcaatcta tcgtcgttcc 1380
gacctagtaa gggagcaggt gccagaatcg tctcaaaggc aattcgcagc atgttcagaa 1440
tctgaaacga ggcctgtgca atggtttact gcttctgggt cagatgcaaa caatacggaa 1500
aatatcaccg ccagcgatcc cgtcgatcgc acgggtggag ttaagcggct aggctccaaa 1560
agcgacagaa ccgttacagc ttctatccat gacgtgaatt ccagcacaag gcgactgttg 1620

cttaacgaat ttggatcgga ggctccgcgc ccttcgccag aaaagactgt tcgcttaaga 1680
agcgacaata ttggcaccta tgggagccgg aaaaatgaac gagcgcggct cgcgaccgaa 1740
accggtgcgt atgagtcgga gcatattttc gggttcaagg ctgtccacga tactgcgaga 1800
gcgacgaaag agggccggcg tctcgaaagg cccatgcccg cctaccttga ggataagggg 1860
cttcatcgcc aacatattgg caccgggaga ggacggacca aacttgtcgg gcgcggatgg 1920
ccggatgaca caagctatcg ctcggatcaa agggcaactc tgtcggaccc cgttgcgcgc 1980
tcggaaggcg cgacggcctc aaatgggtat caattgaacc aattgggtta cgcgcaccaa 2040
ctcgctagcg atggcctgca aagtgaatcg cccgatggtg ttgccttgcc aattcaagtg 2100
gcaacaacga gctacaacta tacagtgagc cgcgatcctg tccttgttcc gccggataaa 2160
aacgaagccc ctcaattgct gcatcttggt ccccgtggtc aaaccgaagc tgttcttgcc 2220
cgcgaaacag cattgactgg aaaatggccg actctcgagc gtgagcagca agtgtatcgc 2280
gagtttttgg ccttatatga cgtaaaaaaa gatcttgagg ccaaatcagt cggcgtaaga 2340
cggaaaaaaa aagaagttat ttctgcgtta gaccgaactg cgcgcttgat aagcacgtcg 2400
ccttcgaaag ctcgatccaa agcagagact gaaaaagcca ttgatgagct cgatgatcga 2460
cgagtttatg atccgcgtga tcgagctcaa gacaaagcgt ttaaacgctg a 2511
<210> 21
<211> 198
<212> DNA
<213> Agrobacterium EHA101
<400> 21
atggccatca tcaagccgca tgtgaacaaa aataggacaa cctcgccgat agagagaccg 60
gagtctctca tagaggaaat gagcggcagt catccgccga gtggttttac caacctggat 120
ctcgctatga tcgagctgga ggactttgtc catcggtgcc cgctcccaga agacaatctt 180
gctggtcaga aggagtga 198
<210> 22
<211> 1650
<212> DNA
<213> Agrobacterium EHA101
<400> 22
   atggatccgt ctagcaatga gaatgtctat gtgggtcgcg gtcacaacat cgaaaatgat 60
   gatgacactg accccaggcg ttggaagaag gcgaatatca gttccaacac catctccgat 120
   attcagatga cgaatggcga agacgtacaa tcagggagcc ctacccgaac ggaagttgta 180
   agcccacgtc tggattatgg atcggtcgac tcctcctcca gcctttattc tggcagcgag 240
   cacggaaatc aagctgagat tcaaaaagag ctgtccgtct tgttctcgaa catgtctttg 300
   ccaggcaacg atcggcgccc ggacgaatac attctcgtgc atcaaacggg acaagatgct 360
   tttactggta ttgccaaagg caacctcgac caaatgccca ccaaggcgga atttaacgcg 420
   tgctgccgtc tctacaggga cggagccggt aattactacc cgccacctct cgcattcgac 480
   aagattagcg ttccagagca actggaggaa aaatggggga tgatggaggc gaaggaacgt 540
   aacaaactgc ggtttcagta caagttggac gtatggaatc atgcgcacgc tgatatgggg 600
   atcacgggca cagagatctt ttatcaaaca gataagaaca taaagctcga ccggaattat 660
   aaactaagac ctgaagaccg atacgtacaa acagaaaaat acgggcgccg ggaaattcaa 720
   aagcgatatc aacacgaact ccaggctggt tcgctgctgc ccgatattat gatcaaaact 780
   ccccaaaatg acatccactt cgtgtacagg tttgccggcg acaattacgc caacaaacag 840
   ttcagcgagt ttgaacacac cgtcaagcgc aggtatggcg acgagactga gatcaaattg 900
   aagtcaaagt caggcattat gcatgactcg aaatatctgg aatcctggga acggggcagt 960
   gcggatattc gcttcgcgga attcgttggg gaaaatagag ctcacaatcg gcagtttcca 1020
   actgcgacag taaatatggg acagcagcca gacgggcagg gcggtttgac ccgcgaccgt 1080
   catgtgagcg ttgacttcct aatgcaaagc gcacccaatt cgccttgggc gcaagctttg 1140
   aaaaagggag aactgtggga tcgcgttcag ttgcttgctc gcgacggcaa ccgctatctg 1200
   tcgccgccca gattggaata ttctgaccct gcacatttca ccgagttgat gaaccgggtt 1260
   ggtttacccg catcgatggg tcggcaaagc catgcggcta gtatcaaatt cgaaaagttt 1320
   gacgcgcagg cagcggttat tgtcttaaat ggcccagagt tacgtgacat tcatgacttg 1380
   tctcctgaaa aactgcaaaa tttgtccacc aaagatgtca tcgtcgccga tcgcaatgag 1440
   aatggtcaga gaactggcac gtacaccagc gtcgcggaat atgagcgctt gcagttaagg 1500
   ctgccacccg atgcagcggg ggtgcttggt gaagcaactg acaaatattc acgtgatttc 1560
   gttcggccag agccggcgtc gcgtccaatc agtgacagcc gcaggatata cgaaagtcga 1620
   ccgcgtagcc aaagcgtcaa cagcttttga 1650
<210> 23

<211>  2019
<212>  DNA
<213>  Agrobacterium  EHA101
<400>  23
   atggtgaaca ctacaaagaa aagttttgcg aagtcgctta cggcagatat gcgccgttct      60
   gctcagcgcg ttgtcgagca aatgcgaaaa gcattgatta ccgaagaaga ggcgctcaag     120
   cggcaagcca gactggagag tcccgatagg aagcgaaagt atgctgctga tatggcgata     180
   gtcgacaaac tcgacgtagg gtttcgaggc gaaataggct ataaaattct tggaaataac     240
   cggcttcgag tagacaacca taaagaatta acgcgtgagc acggtagact tcgcaaaacc     300
   aaaacggttc tgaagcgtaa cccggtgacg caggaagtct atttgggttt atatgaaagg     360
   aagtcctggt taagtgtcag cagccatttg tatgctgcgg acggcacact ccgcatgaag     420
   cacgtgaaat acaaagacgg acgttttgag gaaaaatggg agcgcgacga aaatggcgac     480
   ctgatccgca caaggtacgc caaccgtggc aggctctttc aacctgtatc cgagaaaatg     540
   ggcgcgccgt atcggagcgg ccctgacgac cggctctatc gcgatctaac ccgtcgaaac     600
   ggtttcagac gggagacatt cgaacgggac gatcacggaa acctcgagcg tatcggcagc     660
   aaccatgtcg gcttttccaa gatttcagtg aaggcaccca atcgtcaaac ctcccagacg     720
   aagattcaaa aacttggtgg cgctttcaac aaatctttta ggtcccttct ggacaaggag     780
   ggcaatgaaa tgggccgcga tattttgagc catcgacggc tctataacaa gcggtctgct     840
   gtctacgatg aagctaccgg acaattgaag agtgccaagc ataccttcgg caagatctac     900
   aggagcgaaa ccgaatatct cagcgcgggc ctcaagaagg tttcaaaaaa gatactcggg     960
   gtgacggtct accggaaatt tgcggcgctc agcgagcgag aatccgaggc tgagagactg    1020
   cgtagttttg aatccggtgc gcatcgccag atctggcagg agcgggcagc gattcccggt    1080
   tcgcccctcc cggagactga tgacattcat ttcgcacagc agtcgcacct agccaaagcc    1140
   aaccctgatc acgtcgaagc tgacgtcacg cgtgtgacag atcaacatgt tgatgttgct    1200
   ggacaaacat catcgtctcc ccaacggaac ttggaaggat ggttagattc tcaatcacga    1260
   tacaagccag caaacatgct gttgtcaaat ccagaccttc aagcgaacgg acctcgccca    1320
   tacgaagggt tagctcatct caccctccgg cgcgataatg aatctgacgg gcacaaggag    1380
   aatgatcagc ggctgcgaca tttctcccag ccagagccgt tggtgttacc gcatcccggg    1440
   tcgccggaaa taactaaggt gtttggctcg cggggagagc cggcacaccc gagtggaaat    1500
   ctgcacacgg cggttggaga aacggcttgc gaaggaccgg tgatgtcttc atcctcggac    1560
   aatcatcagc cagctccagg acagcaagaa cttttaagtt tccttcataa tgcgccagcc    1620
   ccagtttctg tggcaataca tgatgatcaa gagcgacttg cgggggaggc gcccggcggc    1680
   tctttcagag gtagctcagg gcgaacgagt tcaatgtcgg agagtatctt cgacgaagat    1740
   gtacaagggc atttggtacg ggattattcg atcaatacta ctaacgggtt tattgacccg    1800
   caatcgttgt tcggtgaacc ggacttatcg agaggtccaa aatcggggcc agaaattcca    1860
   tcggaagatt accatttgtc agcttcggaa caggaaaatt tgctgaatca attgcttagt    1920
   gtgccactgc cggttccttc accgaagccc gaatgcgcga ggtctatgat tttcgaaggt    1980
   tcacgttcaa gagagcgttc cacctccaga gggttctaa                           2019
<210>  24
<211>  438
<212>  DNA
<213>  Agrobacterium  EHA101
<400>  24
atgaaacgta tcagtacaat ccttgtcggc gtatttttgg ctgcgccggt ttatgcagcc      60
gacaatattc atacattagg gacgttgtct gaaattgaac tagcgctcac tgccggaaag     120
ccggtgaacg tcacggttga tttaagtttg tgtgctcccg gggtggccga tactccggcg     180
acgaaaacgc aaggaggcat gcgcatagat gcgtatcgaa taacaactga cggcaccctt     240
gcgtttgcag atcagcactt caccattgac cgtgacggta aacccataac tcaatttatt     300
cgttatcaga ttcggtcaaa cggtgacgcc gatttcacga tggttacatt caacatgcca     360
acatatgagc gaaaaggtac cagcttggcc tacaagtgcg cgatcgacca cgggttgagt     420
tttcgtactc cacagtga                                                   438
<210>  25
<211>  696
<212>  DNA
<213>  Agrobacterium  EHA101
<400>  25
tcaagcctcc aagattttgc tgatcagttt cgaaatgacc acgacttcct ccatcgcaat      60
ccgaagattc ctctctatga ggcgcatcgt cggatcagtt cccgtgttta gtaatgtaag     120

atgcaacatg ccgcgttctt tcatcgcggc aaatgcatct ctttcatgca tgggagacgg 180
tacaactgga aggctctcta gcgtctctga catcctgcgt tgcgatgttg tcaatcggcc 240
gaccgggacg cgttggcgca aaacagctgt aggaattgcc aaattttcac tcaacagcag 300
ctcgatgacg tagcggtagg tagatagtgc ctcatcgatg tcgagcggcg ttagcatggt 360
ggggatcaga agcaggtttg agctagcgat gattgtgttg ttgagctcgc tcgagccgcc 420
acgcgtatcg gccaacgcat aatcaaatcc ttcgagctcg gcattttcat aggctgcttc 480
aagaaggggc atttcgtcgg cggaatagac ttcacagcga ggatcccagg tactgctttg 540
taaggcgttt tctctccatc gcgtcagagg ccggttttcg tcggcatcaa agagggccac 600
tcgtttaccg tcatttgcca aagcagcgca aaggcccatg agtgcggtgg ttttgccagc 660
accccctttg aaagaacaaa acgtcaaaag ttgcat 696
<210> 26
<211> 20
<212> DNA
<213> Artificial Sequence
<220>
<223> Description of Artificial Sequence: Synthetic DNA
<400> 26
ttagcgcctc tgaaattcgg 20
<210> 27
<211> 21
<212> DNA
<213> Artificial Sequence
<220>
<223> Description of Artificial Sequence: Synthetic DNA
<400> 27
aaggcacttc gtcagcgacc a 21
<210> 28
<211> 22
<212> DNA
<213> Artificial Sequence
<220>
<223> Description of Artificial Sequence: Synthetic DNA
<400> 28
ttacttctga ccgcaagaga tc 22
<210> 29
<211> 20
<212> DNA
<213> Artificial Sequence
<220>
<223> Description of Artificial Sequence: Synthetic DNA
<400> 29
ttcgccagat gctcagcgat 20
<210> 30
<211> 21
<212> DNA
<213> Artificial Sequence
<220>
<223> Description of Artificial Sequence: Synthetic DNA
<400> 30
ggtggttgga aagctcatgt c 21
<210> 31
<211> 20
<212> DNA
<213> Artificial Sequence
<220>
<223> Description of Artificial Sequence: Synthetic DNA
<400> 31
cgatccagaa aatcctggcg 20

<210> 32
<211> 20
<212> DNA
<213> Artificial Sequence
<220>
<223> Description of Artificial Sequence: Synthetic DNA
<400> 32
caccggcttc tgcgacgcgc                    20
<210> 33
<211> 22
<212> DNA
<213> Artificial Sequence
<220>
<223> Description of Artificial Sequence: Synthetic DNA
<400> 33
aatctatccg tagatcatcc tc                 22
<210> 34
<211> 19
<212> DNA
<213> Artificial Sequence
<220>
<223> Description of Artificial Sequence: Synthetic DNA
<400> 34
gtggatatcg aggggacac                     19
<210> 35
<211> 20
<212> DNA
<213> Artificial Sequence
<220>
<223> Description of Artificial Sequence: Synthetic DNA
<400> 35
acatactttc atcgaagtcg                    20
<210> 36
<211> 20
<212> DNA
<213> Artificial Sequence
<220>
<223> Description of Artificial Sequence: Synthetic DNA
<400> 36
ggtcaccgtt ccgttcggtt                    20
<210> 37
<211> 21
<212> DNA
<213> Artificial Sequence
<220>
<223> Description of Artificial Sequence: Synthetic DNA
<400> 37
tctccgcgaa cagatctact g                  21
<210> 38
<211> 20
<212> DNA
<213> Artificial Sequence
<220>
<223> Description of Artificial Sequence: Synthetic DNA
<400> 38
caattgactg acctcgcgaa                    20
<210> 39
<211> 20

<212> DNA
<213> Artificial Sequence
<220>
<223> Description of Artificial Sequence: Synthetic DNA
<400> 39
cgtgcatgct ccttctttct                    20
<210> 40
<211> 20
<212> DNA
<213> Artificial Sequence
<220>
<223> Description of Artificial Sequence: Synthetic DNA
<400> 40
ttcctggatc caccgcgctg                    20
<210> 41
<211> 20
<212> DNA
<213> Artificial Sequence
<220>
<223> Description of Artificial Sequence: Synthetic DNA
<400> 41
atcggctcaa ttgatatccc                    20
<210> 42
<211> 20
<212> DNA
<213> Artificial Sequence
<220>
<223> Description of Artificial Sequence: Synthetic DNA
<400> 42
tttgcttggt cacctgaaac                    20
<210> 43
<211> 20
<212> DNA
<213> Artificial Sequence
<220>
<223> Description of Artificial Sequence: Synthetic DNA
<400> 43
ccgcacttac gtcctcgtac                    20
<210> 44
<211> 22
<212> DNA
<213> Artificial Sequence
<220>
<223> Description of Artificial Sequence: Synthetic DNA
<400> 44
agttgaggat gtttttcagg ag                 22
<210> 45
<211> 21
<212> DNA
<213> Artificial Sequence
<220>
<223> Description of Artificial Sequence: Synthetic DNA
<400> 45
tagggtcggt tactctagga t                  21
<210> 46
<211> 20
<212> DNA
<213> Artificial Sequence

<220>
<223> Description of Artificial Sequence: Synthetic DNA
<400> 46
aaaccgcact cccgatattc                    20
<210> 47
<211> 20
<212> DNA
<213> Artificial Sequence
<220>
<223> Description of Artificial Sequence: Synthetic DNA
<400> 47
cactcggtcc ttctctatct                    20
<210> 48
<211> 20
<212> DNA
<213> Artificial Sequence
<220>
<223> Description of Artificial Sequence: Synthetic DNA
<400> 48
aggctgtgtt gttcgcagca                    20
<210> 49
<211> 22
<212> DNA
<213> Artificial Sequence
<220>
<223> Description of Artificial Sequence: Synthetic DNA
<400> 49
ccaaagattg gccccttgat ac                 22
<210> 50
<211> 20
<212> DNA
<213> Artificial Sequence
<220>
<223> Description of Artificial Sequence: Synthetic DNA
<400> 50
gttggttccc aatgccaatc                    20
<210> 51
<211> 20
<212> DNA
<213> Artificial Sequence
<220>
<223> Description of Artificial Sequence: Synthetic DNA
<400> 51
cacttcaccg agattcttcg                    20
<210> 52
<211> 20
<212> DNA
<213> Artificial Sequence
<220>
<223> Description of Artificial Sequence: Synthetic DNA
<400> 52
cttcaagctg cctttcacat                    20
<210> 53
<211> 20
<212> DNA
<213> Artificial Sequence
<220>
<223> Description of Artificial Sequence: Synthetic DNA

<400> 53
aagatcaaag gcgactcctc                    20
<210> 54
<211> 20
<212> DNA
<213> Artificial Sequence
<220>
<223> Description of Artificial Sequence: Synthetic DNA
<400> 54
gcctttcaca ttggaatcat                    20
<210> 55
<211> 20
<212> DNA
<213> Artificial Sequence
<220>
<223> Description of Artificial Sequence: Synthetic DNA
<400> 55
atgggaagac ctcgttgtca                    20
<210> 56
<211> 20
<212> DNA
<213> Artificial Sequence
<220>
<223> Description of Artificial Sequence: Synthetic DNA
<400> 56
aaggtcgtcc acgatacttt                    20
<210> 57
<211> 20
<212> DNA
<213> Artificial Sequence
<220>
<223> Description of Artificial Sequence: Synthetic DNA
<400> 57
ggcactgctg tcaagaaatc                    20
<210> 58
<211> 20
<212> DNA
<213> Artificial Sequence
<220>
<223> Description of Artificial Sequence: Synthetic DNA
<400> 58
attcctgagc attgaggtcc                    20
<210> 59
<211> 20
<212> DNA
<213> Artificial Sequence
<220>
<223> Description of Artificial Sequence: Synthetic DNA
<400> 59
tgctttctag gctgctgcag                    20
<210> 60
<211> 20
<212> DNA
<213> Artificial Sequence
<220>
<223> Description of Artificial Sequence: Synthetic DNA
<400> 60
atggagcaaa ccttaatttg                    20

<210> 61
<211> 20
<212> DNA
<213> Artificial Sequence
<220>
<223> Description of Artificial Sequence: Synthetic DNA
<400> 61
ggacatcccg gtggaattta                    20
<210> 62
<211> 20
<212> DNA
<213> Artificial Sequence
<220>
<223> Description of Artificial Sequence: Synthetic DNA
<400> 62
accgaaggct caattctatt                    20
<210> 63
<211> 20
<212> DNA
<213> Artificial Sequence
<220>
<223> Description of Artificial Sequence: Synthetic DNA
<400> 63
atcattgttt ctcctacaga                    20
<210> 64
<211> 20
<212> DNA
<213> Artificial Sequence
<220>
<223> Description of Artificial Sequence: Synthetic DNA
<400> 64
aaggagttag acgatggatc                    20
<210> 65
<211> 19
<212> DNA
<213> Artificial Sequence
<220>
<223> Description of Artificial Sequence: Synthetic DNA
<400> 65
cagctcgcat tgaattccg                     19
<210> 66
<211> 19
<212> DNA
<213> Artificial Sequence
<220>
<223> Description of Artificial Sequence: Synthetic DNA
<400> 66
acctggcggc aaaccgaca                     19
<210> 67
<211> 20
<212> DNA
<213> Artificial Sequence
<220>
<223> Description of Artificial Sequence: Synthetic DNA
<400> 67
tagccggcta ggttttcttc                    20
<210> 68
<211> 19

<212> DNA
<213> Artificial Sequence
<220>
<223> Description of Artificial Sequence: Synthetic DNA
<400> 68
ggtcgacacg acgaagaag                    19
<210> 69
<211> 20
<212> DNA
<213> Artificial Sequence
<220>
<223> Description of Artificial Sequence: Synthetic DNA
<400> 69
tatttcacat gcttcgcgcg                    20
<210> 70
<211> 20
<212> DNA
<213> Artificial Sequence
<220>
<223> Description of Artificial Sequence: Synthetic DNA
<400> 70
accgcgtcag tgacgaagcc                    20
<210> 71
<211> 20
<212> DNA
<213> Artificial Sequence
<220>
<223> Description of Artificial Sequence: Synthetic DNA
<400> 71
gcgttacgct tcagaacctt                    20
<210> 72
<211> 19
<212> DNA
<213> Artificial Sequence
<220>
<223> Description of Artificial Sequence: Synthetic DNA
<400> 72
tgaaacaggc caagatgtg                    19
<210> 73
<211> 20
<212> DNA
<213> Artificial Sequence
<220>
<223> Description of Artificial Sequence: Synthetic DNA
<400> 73
tcattcacca tcgcgcgcca                    20
<210> 74
<211> 20
<212> DNA
<213> Artificial Sequence
<220>
<223> Description of Artificial Sequence: Synthetic DNA
<400> 74
atcaattgag ccgatcttcc                    20
<210> 75
<211> 20
<212> DNA
<213> Artificial Sequence

<220>
<223> Description of Artificial Sequence: Synthetic DNA
<400> 75
aggtgatcct atttagattg                    20
<210> 76
<211> 19
<212> DNA
<213> Artificial Sequence
<220>
<223> Description of Artificial Sequence: Synthetic DNA
<400> 76
tgatctctga ctcctgtgt                     19
<210> 77
<211> 20
<212> DNA
<213> Artificial Sequence
<220>
<223> Description of Artificial Sequence: Synthetic DNA
<400> 77
cttccatgtg ctgatttcca                    20
<210> 78
<211> 20
<212> DNA
<213> Artificial Sequence
<220>
<223> Description of Artificial Sequence: Synthetic DNA
<400> 78
tctctgcgtc  gatttcaaga                20
<210> 79
<211> 20
<212> DNA
<213> Artificial Sequence
<220>
<223> Description of Artificial Sequence: Synthetic DNA
<400> 79
tccttatcct  gtcgattttg                   20
<210> 80
<211> 20
<212> DNA
<213> Artificial Sequence
<220>
<223> Description of Artificial Sequence: Synthetic DNA
<400> 80
cagatgggaa  ttcgcaaacc                   20
<210> 81
<211> 20
<212> DNA
<213> Artificial Sequence
<220>
<223> Description of Artificial Sequence: Synthetic DNA
<400> 81
tgtgtgaggg  atcggataac                   20
<210> 82
<211> 20
<212> DNA
<213> Artificial Sequence
<220>
<223> Description of Artificial Sequence: Synthetic DNA

<400> 82
ctacgcgcat gaagatcaag                    20
<210> 83
<211> 20
<212> DNA
<213> Artificial Sequence
<220>
<223> Description of Artificial Sequence: Synthetic DNA
<400> 83
tcctcgccct tgctcaccat                    20